# FASCICULE DE BREVET EUROPEEN

(11) **EP 0 927 173 B1**
(45) Date de publication et mention de la délivrance du brevet: **12.06.2002**
(21) Numéro de dépôt: 97915205.5
(22) Date de dépôt: 28.03.1997
(51) Int. Cl.: C07D 295/08, C07D 295/20

(54) **[2-(1-PIPERAZINYL)ETHOXY]METHYLE SUBSTITUES**
SUBSTITUIERTE [2-(1-PIPERAZINYL)ETHOXY]METHYL-DERIVATE
SUBSTITUTED [2-(1-PIPERAZINYL)ETHOXY]METHYL

(30) Priorité: 10.04.1996 BE 9600310
(43) Date de publication de la demande: 07.07.1999
(73) Titulaire: UCB, S.A., 1070 Bruxelles (BE)
(72) Inventeur: DUCHENE, Guy, B-1933 Sterrebeek (BE); DELEERS, Michel, B-1630 Linkebeek (BE); BODSON, Guy, B-6730 Bellefontaine (BE); MOTTE, Geneviève, B-1450 Chastre (BE); LURQUIN, Françoise, B-1495 Villers-la-Ville (BE)
(86) Numéro de dépôt international: BE9700038
(87) Numéro de publication internationale: WO9737982

(56) Documents cités:
- EP-A- 0 058 146
- GB-A- 1 174 819
- GB-A- 1 184 395
- GB-A- 2 225 320
- GB-A- 2 225 321

## Description

La présente invention se rapporte à des composés [2-(1-pipérazinyl)éthoxy]méthyle substitués de formule 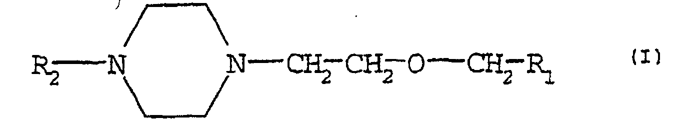 dans laquelle
R₁ représente un groupe -CONH₂, -CN, -COOH, -COOM ou -COOR₃, M étant un métal alcalin et R₃ étant un radical alkyl ayant de 1 à 4 atomes de carbone; et
R₂ représente un atome d'hydrogène ou un groupe -COR₄ ou -R₅, où R₄ est choisi parmi les groupes -OR₆ ou -R₇, dans lesquels
R₅ représente un radical allyle ou alkylaryle,
R₆ représente un radical alkyle linéaire ou ramifié ayant de 1 à 4 atomes de carbone, un radical halogénoalkyle, alkylaryle, alkylnitroaryle ou alkylhalogénoaryle, et
R₇ représente un halogénoalkyle,
à un procédé de préparation de ces composés, de même qu'à leur utilisation pour la préparation de composés de formule 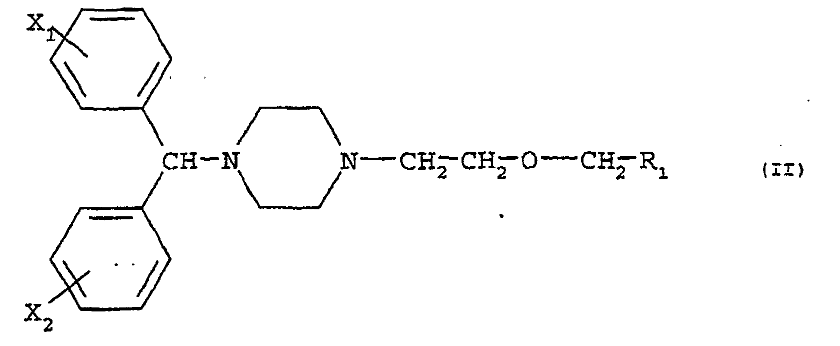 dans laquelle R₁ a la même signification que dans la formule I et X₁ et X₂ représentent indépendamment un atome d'hydrogène, de fluor, de chlore ou de brome. Dans le cas où R₁ représente un groupe -COOH et où X₁ représente un atome de chlore en position 4 et X₂ représente un atome d'hydrogène, le composé de formule II est l'acide 2-[2-[4-[(4-chlorophényl)phénylméthyl]-1-pipérazinyl]éthoxy]acétique de formule 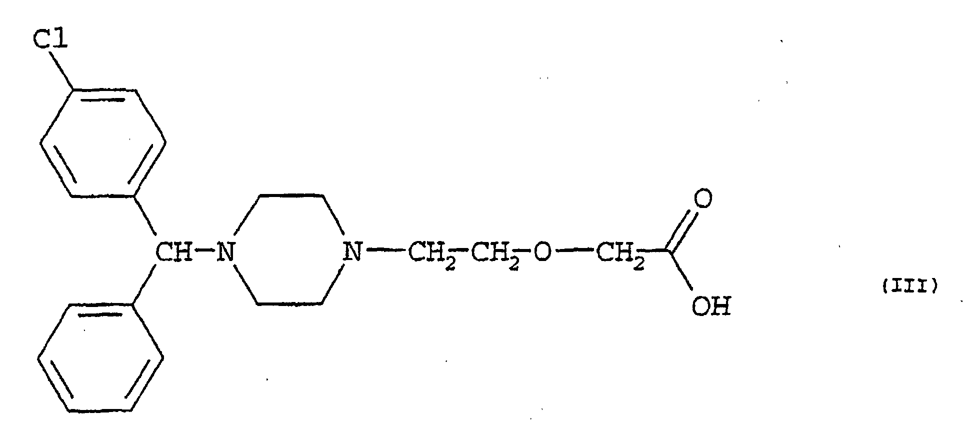 Dans le cas où R₁ représente un groupe -CONH₂, -CN, -COOM ou -COOR₃, M étant un métal alcalin et R₃ étant un radical alkyle ayant de 1 à 4 atomes de carbone et où X₁ représente un atome de chlore en position 4 et x₂ un atome d'hydrogène, les composés de formule II sont des produits intermédiaires précieux pour la préparation de l'acide 2-(2-[4-[(4-chlorophényl)phénylméthyl]-1-pipérazinyl]éthoxy]acétique de formule III ainsi que de ses sels pharmaceutiquement acceptables.

Composés de formule II dans laquelle R₁ représente -CH₂X X étant un atome d'halogène ou un groupe -OH ont été décrits dans GB-A-1 174 819.
Le dichlorhydrate de l'acide 2-[2-[4-[(4-chlorophényl)phénylméthyl]-1-pipérazinyl]éthoxy]acétique, produit qui est également connu sous la dénomination commune internationale de cétirizine, est décrit dans le brevet canadien 1.199.918 et a été introduit comme médicament pour le traitement des syndromes allergiques, comme la rhinite allergique chronique et aiguë, la conjonctivite allergique, le prurit, l'urticaire, etc. Dans son application thérapeutique, ce produit s'est avéré remarquablement dépourvu d'effets secondaires sur le système nerveux central tels que la somnolence, la performance mentale atténuée, etc. (cfr. D.P. TASHKIN et al., Annals of Allergy, Part II, 59, (1987), 49-52, ainsi que F.M. GENGO et al., Annals of Allergy, Part II, 59, (1987),53-57).

Dans le cas où R₁ représente un groupe -COOH et où X₁ et X₂ représentent chacun un atome de fluor en position 4, le composé de formule II est l'acide 2-[2-(4-[bis(4-fluorophényl)méthyl]-1-pipérazinyl]éthoxy]acétique de formule 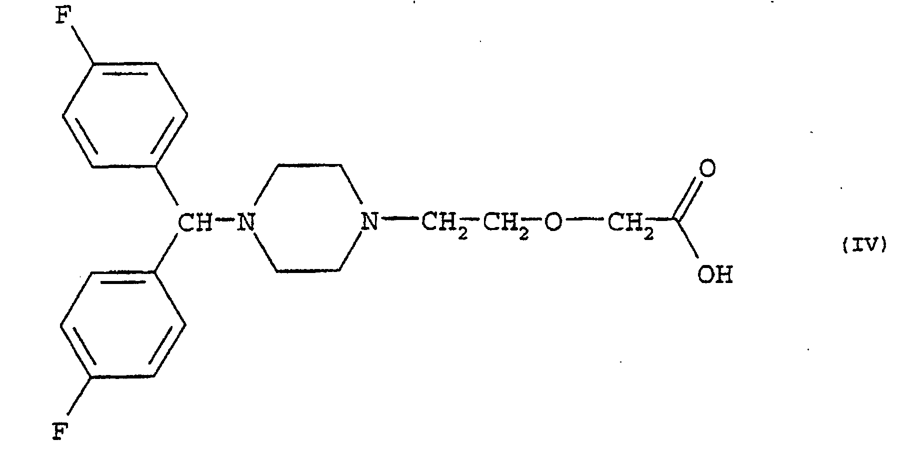 Dans le cas où R₁ représente un groupe -CONH₂, -CN, -COOM ou -COOR₃, M étant un métal alcalin et R₃ étant un radical alkyle ayant de 1 à 4 atomes de carbone et où X₁ et X₂ représentent chacun un atome de fluor en position 4, les composés de formule II sont des produits intermédiaires précieux pour la préparation de l'acide 2-[2-[4-[bis(4-fluorophényl)méthyl]-1-pipérazinyl]éthoxy]acétique de formule IV ainsi que de sels pharmaceutiquement acceptables.

Le dichlorhydrate de l'acide 2-[2-[4-[bis(4-fluorophényl)méthyl]-1-pipérazinyl]éthoxy]acétique est également connu sous la dénomination commune internationale d'éflétirizine. L'utilisation de l'éflétirizine pour le traitement de rhinites ou de rhino-conjonctivites d'origine allergique a été suggérée au cours de plusieurs communications récentes (51^{th} Annual Meeting or American Academy of Allergy and Immunology, reproduit dans J. Allergy Clin. Immunol., 95/1 (1995), part 2, Abstract 229 et XV^{th} Congress of Allergology and Clinical Immunology, reproduit dans Allergy & Clin. Immunol. News, (1994) suppl. n°2, abstracts 428, 1136, 1496 et 1864). Ces communications indiquent que l'administration intranasale d'éflétirizine pourrait fournir un traitement thérapeutique efficace des rhinites ou rhino-conjonctivites d'origine allergique.

Le brevet canadien 1.199.918 au nom de la demanderesse, décrit la synthèse de l'acide 2-[2-[4-[(4-chlorophényl)phénylméthyl]-1-pipérazinyl]éthoxy]acétique et de son dichlorhydrate. Dans cette synthèse, le produit de départ est la 1-[(4-chlorophényl)phénylméthyl]pipérazine, que l'on peut faire réagir avec le (2-chloroéthoxy)acétate de méthyle ou le 2-(2-chloroéthoxy)acétamide pour former respectivement le 2-[2-[4-[(4-chlorophényl)phénylméthyl]-1-pipérazinyl]éthoxy]acétate de méthyle (composé de formule II avec R₁ = -COOCH₃, X₁ = -Cl (position 4) et X₂ = -H) ou le 2-[2-[4-[(4-chlorophényl)phénylméthyl]-1-pipérazinyl)éthoxy]acétamide (composé de formule II avec R₁ = -CONH₂, X₁ = -Cl (position 4) et X₂ = -H). On peut ensuite soumettre cet ester méthylique ainsi que cet acétamide à une hydrolyse avec une base minérale (hydroxyde de potassium ou de sodium) pour former le sel sodique ou potassique que l'on transforme facilement en cétirizine et en son dichlorhydrate.

Le brevet canadien 1.320.732 également au nom de la demanderesse fournit une voie de synthèse alternative pour la préparation de l'acide 2-[2-[4-[(4-chlorophényl)phénylméthyl]-1-pipérazinyl]éthoxy]acétique et de son dichlorhydrate.

Selon ce brevet, on prépare l'acide 2-[2-[4-[(4-chlorophényl)phénylméthyl]-1-pipérazinyl]éthoxy]acétique et son dichlorhydrate par un procédé qui est caractérisé en ce que l'on fait réagir le 2-[4-[(4-chlorophényl)phénylméthyl]-1-pipérazinyl]éthanol avec un halogénoacétate de métal alcalin, en présence d'un alcoolate de métal alcalin, et en ce que l'on transforme le sel de métal alcalin ainsi obtenu en l'acide correspondant et. le cas échéant, en son dichlorhydrate.

Le brevet canadien 1.317.300, également au nom de la demanderesse apporte un autre procédé de synthèse qui permet de préparer l'acide 2-[2-[4-[(4-chlorophényl)phénylméthyl]-1-pipérazinyl]éthoxy]acétique et son dichlorhydrate.

Selon ce brevet, on prépare l'acide 2-[2-[4-[(4-chlorophényl)phénylméthyl]-1-pipérazinyl]éthoxy]acétique et son dichlorhydrate par un procédé qui est caractérisé en ce que l'on hydrolyse le 2-[2-[4-[(4-chlorophényl)phénylméthyl]-1-pipérazinyl)éthoxy]acétonitrile (composé de formule II avec R₁ = -CN, X₁ = -Cl (position 4) et X₂ = -H) en milieu aqueux, alcoolique ou hydro-alcoolique et par une base ou par un acide, et en ce que l'on transforme l'acide ainsi obtenu, le cas échéant, en son dichlorhydrate.

Le 2-[2-[4-[(4-chlorophényl)phénylméthyl]-1-pipérazinyl]éthoxy]acétonitrile utilisé comme produit de départ est obtenu en faisant réagir la 1-[(4-chlorophényl)phénylméthyl]-pipérazine avec un 2-halogénoéthoxyacétonitrile. Cette réaction est conduite en présence d'un accepteur d'acide, tel qu'un carbonate de métal alcalin, et éventuellement en présence d'une faible quantité d'iodure d'un métal alcalin pour accélérer la réaction, dans un solvant organique inerte tel qu'un alcool (par exemple le n-butanol, etc.), de préférence à une température voisine de la température de reflux. Etant donné l'intérêt thérapeutique croissant de la cétirizine et des composés de structure voisine, la demanderesse s'est fixé comme objectif et a entrepris des travaux de recherche dans le but de mettre au point une nouvelle voie de synthèse pour l'acide 2-[2-[4-[(4-chlorophényl)phénylméthyl]-1-pipérazinyl]éthoxy]acétique et de ses sels pharmaceutiquement acceptables qui permettrait d'obtenir ce composé au départ de réactifs connus et/ou facilement accessibles et qui en outre, fournirait ce composé avec une pureté suffisante et un rendement économiquement acceptable. D'autre part, en vue de simplifier l'industrialisation du procédé, la demanderesse s'est fixé comme autre objectif de mettre au point une voie de synthèse dont le nombre d'étapes pourrait être réduit par rapport aux procédés connus.

En outre, étant donné l'intérêt thérapeutique d'autres composés de formule II, tels que l'éflétirizine par exemple, il serait intéressant que ces autres composés puissent être préparés selon des procédés sensiblement similaires. Par voie de conséquence, il est nécessaire de trouver des précurseurs qui, d'une part peuvent eux-mêmes être préparés de manière aisée et économique, et d'autre part, peuvent être transformés facilement et avec des rendements élevés en des composés de formule II.

La demanderesse vient de découvrir une famille de composés, en l'occurrence des [2-(1-pipérazinyl)éthoxy]méthyle substitués qui répondent parfaitement à cet objectif.

La présente invention à donc pour objet, en tant que composés nouveaux, des [2-(1-pipérazinyl)éthoxy]méthyle substitués de formule 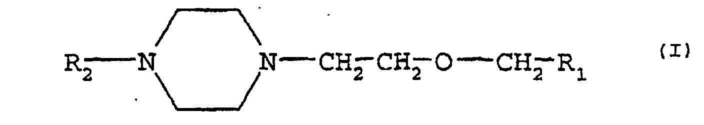 dans laquelle
R₁ représente un groupe -CONH₂, -CN, -COOH, -COOM ou -COOR₃, M étant un métal alcalin et R₃ étant un radical alkyl ayant de 1 à 4 atomes de carbones; et
R₂ représente un atome d'hydrogène ou un groupe -COR₄ ou -R₅, où R₄ est choisi parmi les groupes OR₆ ou R₇, dans lesquels
R₅ représente un radical allyle ou alkylaryle,
R₆ représente un radical alkyle linéaire ou ramifié ayant de 1 à 4 atomes de carbone, un radical halogénoalkyle, alkylaryle, alkylnitroaryle ou alkylhalogénoaryle, et
R₇ représente un halogénoalkyle.

Ces composés peuvent être obtenus aisément par réaction d'une pipérazine de formule 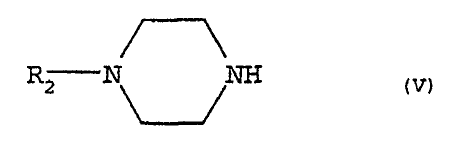 dans laquelle R₂ représente un atome d'hydrogène ou un groupe -COR₄ ou -R₅, où R₄ est choisi parmi les groupes -OR₆ ou -R₇, dans lesquels R₅ représente un radical allyle ou alkylaryle, R₆ représente un radical alkyle linéaire ou ramifié ayant de 1 à 4 atomes de carbone, un radical halogénoalkyle, alkylaryle, alkyinitroaryle ou alkylhalogénoaryle, et R₇ représente un halogénoalkyle,
avec un [2-halogénoéthoxy]méthyle substitué de formule 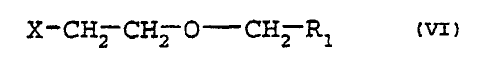 dans laquelle R₁ représente un groupe -CONH₂, -CN, -COOH, -COOM ou -COOR₃, M étant un métal alcalin et R₃ étant un radical alkyle ayant de 1 à 4 atomes de carbone et X représente un atome d'halogène. Le plus souvent, on utilise un composé de formule VI dans laquelle X représente un atome de chlore ou d'iode, mais on peut également mettre en oeuvre cette réaction avec le bromure correspondant. On a constaté que lorsque X représente un atome d'iode, on peut avantageusement travailler à des températures assez basses (inférieures à 40°C) et pendant des temps relativement courts (2 heures par exemple).

Cette réaction est généralement effectuée par chauffage entre 30 et 180°C, pendant plusieurs heures, dans un solvant choisi parmi des alcools aliphatiques, des cétones aliphatiques (par exemple la méthyléthylcétone), des hydrocarbures aromatiques (par exemple le toluène ou le xylène) ou encore dans l'eau et, en présence d'un accepteur d'acide tel qu'une base organique tertiaire (par exemple la triéthylamine) ou une base minérale (par exemple le carbonate de sodium). Lorsque l'on met en oeuvre un large excès de pipérazine (plus de trois équivalents par rapport au (2-halogénoéthoxy]méthyle), la pipérazine elle-même joue le rôle d'accepteur d'acide et il n'est pas indispensable d'ajouter un accepteur d'acide supplémentaire. Avantageusement, on peut également mettre en oeuvre une pipérazine et son dichlorhydrate en quantités équivalentes.

En règle générale, pour préparer les [2-(1-pipérazinyl)éthoxy]méthyle substitués de l'invention, on préfère, pour des raisons évidentes de simplicité, mettre en oeuvre la pipérazine de formule V dans laquelle R₂ représente un atome d'hydrogène. Il peut néanmoins s'avérer judicieux de protéger une des fonctions amine de la pipérazine pendant cette réaction par un groupement protecteur conventionnel de cette fonction afin d'éviter que la pipérazine ne puisse réagir deux fois avec le composé de formule VI.

Comme groupement protecteur de la fonction amine, on peut utiliser tous les groupements protecteurs connus de l'homme du métier pour cet usage.

Il est possible de choisir à cet effet un groupement protecteur qui, après la formation du composé de formule I, peut être clivé sélectivement selon l'équation: 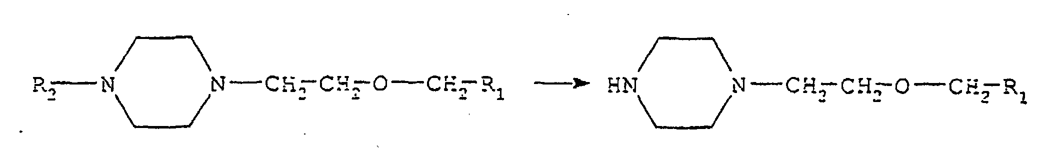 Parmi les méthodes de protection qui conviennent particulièrement bien dans ce cas, on formera par exemple des carbamates d'halogénoalkyle, d'alkylhalogénoaryle, d'alkylaryle, d'alkylnitroaryle, d'alkylhalogénoaryle, des amides, tels que le trifluoroacétamide ou encore des amines tertiaires, telles les N-allylamines ou les N-alkylarylamines.

Avec ces groupements, la réaction de déprotection de la fonction amine de la pipérazine peut être effectuée par simple chauffage, par hydrogénation catalytique ou par hydrolyse au moyen d'une base ou d'un acide, selon des techniques bien connues de l'homme du métier.

Selon une variante, en tant que groupement protecteur de la fonction amine, on peut également choisir un groupement qui peut être clivé dans des conditions telles que, dans le cas où R₁ représente un groupe -CONH₂, -CN, -COOM ou -COOR₃, M étant un métal alcalin et R₃ étant un radical alkyl ayant de 1 à 4 atomes de carbone, simultanément à la réaction de déprotection de la fonction amine, on procède à la conversion du groupe R₁ en un groupe -COOH et, dans le cas où R₁ représente un groupe -COOH, on conserve ce groupe -COOH, lors de la réaction de déprotection de la fonction amine.

Cette réaction s'effectue selon l'équation: 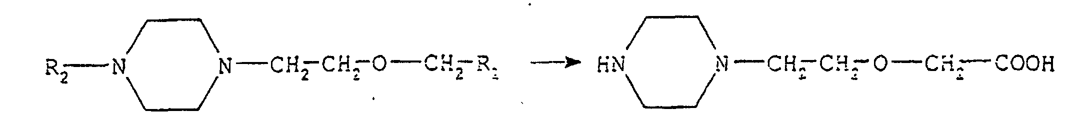 Parmi les méthodes de protection qui conviennent particulièrement bien dans ce cas, on formera par exemple les groupements carbamates d'alkyle.

Avec de tels groupements carboxylés, la déprotection de la fonction amine de la pipérazine peut être effectuée par chauffage pendant plusieurs heures en milieu alcoolique ou aqueux en présence d'une base minérale ou par toute autre méthode conventionnelle connue de l'homme de l'art.
Dans les deux équations qui précèdent,
R₁ représente un groupe -CONH₂, -CN, -COOH, -COOM ou -COOR₃, M étant un métal alcalin et R₃ étant un radical alkyl ayant de 1 à 4 atomes de carbone; et
R₂ représente un atome d'hydrogène ou un groupe -COR₄ ou -R₃, où R₄ est choisi parmi les groupes -OR₆ ou -R₇, dans lesquels
R₅ représente un radical allyle ou alkylaryle,
R₆ représente un radical alkyle linéaire ou ramifié ayant de 1 à 4 atomes de carbone, un radical halogénoalkyle, alkylaryle, alkylnitroaryle ou alkylhalogénoaryle, et
R₇ représente un halogénoalkyle.

Les [2-(1-pipérazinyl)éthoxy]méthyle substitués de formule I ainsi préparés trouvent leur intérêt principal comme précurseurs pour la préparation des composés de formule II (ou des composés ce formule III et IV dans le cas où R₁ représente un groupe -CCOH) qui peuvent ainsi être préparés avec une pureté suffisante, un rendement économiquement acceptable, un nombre d'étapes moindre et à partir des mêmes précurseurs, ce qui représence un avantage non négligeable en ce qui concerne la simplification du procédé industriel et la réduction des coûts de production.

Par voie de conséquence, la présente invention se rapporte également à la préparation de composés de formule II (ou des composés de formule III et IV dans le cas où R₁ représente un groupe -COOH) par réaction d'un composé de formule I dans laquelle R₂ représente un atome d'hydrogène avec un halogénure de diphénylméthyle de formule VII selon l'équation: 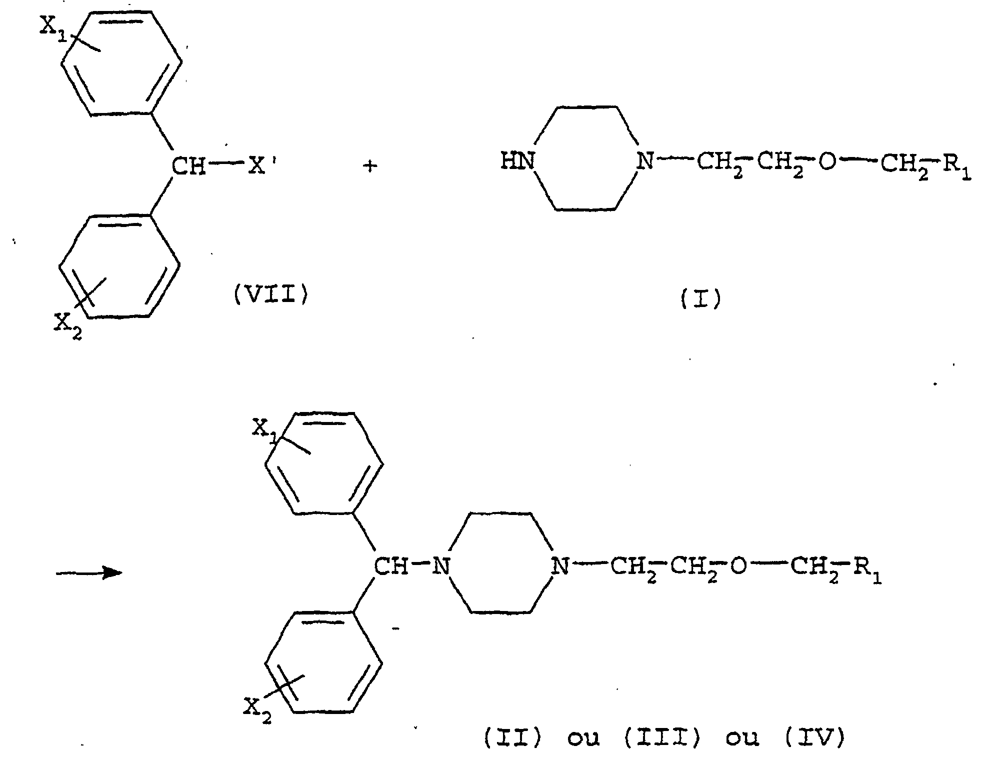 dans laquelle
R₁ représente un groupe -CONH₂, -CN, -COOH. -COOM ou -COOR₃, M étant un métal alcalin et R₃ étant un radical alkyl ayant de 1 à 4 atomes de carbones; et
X' représente un atome d'halogène choisi parmi le chlore, le brome et l'iode,
X₁ et X₂ représentent indépendamment un acome d'hydrogène, de fluor, de chlore ou de brome.

Cette réaction est conduite en faisant réagir l'halogénure de diphénylméthyle de formule VI avec le composé de formule I dans des proportions molaires comprises entre 4:1 et 1:4 pendant un temps compris entre quelques minutes et plusieurs heures à une température comprise entre environ 60 et environ 160°C, dans un solvant inerte choisi parmi des alcools aliphatiques, des cétones aliphatiques (par exemple la méthyléthylcétone), des hydrocarbures aromatiques (par exemple le toluène ou le xylène), des nitriles aliphatiques (par exemple l'acétonitrile). Optionnellement, il est possible de conduire la réaction en présence d'un accepteur d'acide tel qu'une base organique tertiaire (par exemple la triéthylamine) ou une base minérale (par exemple le carbonate de sodium). Eventuellement, cette réaction peut être mise en oeuvre en présence d'un iodure de métal alcalin.

Comme indiqué ci-avant, les composés de formule II (dans laquelle R₁ représente un groupe -CONH₂, -CN, -COOM ou -COOR₃, M étant un métal alcalin et R₃ étant un radical alkyl ayant de 1 à 4 atomes de carbones, X₁ représente un atome de chlore en position 4 et X₂ un atome d'hydrogène) sont déjà connus et leur conversion en l'acide 2-[2-[4-[(4-chlorophényl)phénylméthyl]-1-pipérazinyl]éthoxy]acétique de formule III a déjà été décrite en milieu aqueux, alcoolique ou hydro-alcoolique et par une base ou par un acide. Par ailleurs, le composé de formule II dans laquelle R₁ représente un groupe -COOH et X₁ représente un atome de chlore en position 4 et X₂ un atome d'hydrogène, est l'acide 2-[2-(4-[(4-chlorophényl)phénylméthyl]-1-pipérazinyl)éthoxy]acétique de formule III.

Ainsi donc, pour la conversion ultérieure du 2-[2-[4-[(4-chlorophényl)phénylméthyl]-1-pipérazinyl]éthoxy]acétate d'alkyle (composé de formule II avec R₁ = -COOR₃, X₁ = -Cl en position 4 et X₂ = -H) ou du 2-[2-[4-[(4-chlorophényl)phénylméthyl]-1-pipérazinyl]éthoxyl]acétamide (composé de formule II avec R₁ = -CONH₂, X₁ = -Cl en position 4 et X₂ = -H) en l'acide 2-[2-[4-[(4-chlorophényl)phénylméthyl]-1-pipérazinyl]éthoxy]acétique de formule III, on renvoie au brevet canadien 1.199.918 et pour la conversion du 2-[2-[4-[(4-chlorophényl)phénylméthyl]-1-pipérazinyl]éthoxy]acétonitrile (composé de formule II avec R₁ = -CN, X₁ = -Cl en position 4 et X₂ = -H), on renvoie au brevet canadien 1.317.300. Pour la conversion des autres composés de formule II, on procède par analogie.

Les exemples qui suivent illustrent l'invention. Dans ces exemples, les points de fusion ont été déterminés par calorimétrie à balayage différentiel (D.S.C.) avec un gradient de température de 20°C/min. Les spectres de masse ont été relevés avec un appareil FINNIGAN MAT TSQ 700. Les spectres de résonnance magnétique nucléaire (RMN) ont été relevés avec un appareil Bruker à 250 MHz dans le diméthylsulfoxyde en utilisant le tétraméthylsilane comme étalon interne. Les déplacements chimiques sont indiqués en δ(ppm). Les lettres s, d, dd, t, q, b et m indiquent respectivement un singulet, un doublet, un double doublet, un triplet, un quadruplet, un pic élargi et un multiplet.

### EXEMPLES

### Exemple I. Préparation de [2-(1-pipérazinyl)éthoxy]méthyle substitués de formule I.

### I.1 Préparation des composés de formule I avec R₂ = -H.

### I.1.1. 2-[2-(1-pipérazinyl)éthoxy]acétamide.

### I.1.1.1.

Dans un ballon équipé d'un condenseur refroidi à l'eau et d'un agitateur mécanique, on introduit un mélange de 13,15 g de (2-chloroéthoxy)acétamide (0,1 mole) et de 43 g de pipérazine anhydre (0,5 mole) dans 250 ml de toluène. On chauffe le mélange à la température du reflux pendant 4 heures. On filtre à chaud le précipité qui s'est formé et on évapore le solvant du filtrat sous pression réduite jusqu'à siccité. Le résidu d'évaporation est purifié par chromatographie sur gel de silice (éluant: mélange 14:5:1 (v/v/v) dichlorométhane - méthanol - solution aqueuse d'ammoniac à 28 %). On obtient 7,4 g de 2-[2-(1-pipérazinyl)éthoxy]acétamide sous forme d'une huile jaune.
Rendement: 39 %.
Spectre de masse: 188 (MH⁺), 99 (HN(C₄H₈)N⁺=CH₂), 44 (CONH₂)

### I.1.1.2. (variante).

Dans un ballon à trois cols de 250 ml équipé d'un condenseur refroidi à l'eau et d'un agitateur mécanique, on introduit 8,6 g (0,1 mole) de pipérazine, 15,9 g (0,1 mole) de dichlorhydrate de pipérazine, 10,8 ml (0,6 mole) d'eau et 86 ml de méthyléthylcétone.

On porte la température du mélange à 65°C. On ajoute ensuite en une fois 13,8 g (0,1 mole) de (2-chloroéthoxy)acétamide. On maintient le mélange à la température de 65°C pendant 16 heures. On laisse le mélange revenir à la température ambiante, puis on laisse décanter les deux phases avant de les séparer. On rince la phase inférieure (phase huileuse non miscible avec la néthyléthylcétone) avec 2 x 25 ml de méthyléthylcétone. On reprend cette huile avec 50 ml d'éthanol et on laisse sous agitation pendant 15 minutes.

On filtre le précipité qui s'est formé (dichlorhydrate de la pipérazine) et le filtrat est concentré sous pression réduite à 50°C à l'évaporateur rotatif. On obtient 27 g d'une huile jaune que l'on purifie par chromatographie préparative sur gel de silice (éluant: mélange 82:15:1:2 (v/v/v/v) dichlorométhane/méthanol/solution aqueuse à 28 % (poids) d'ammoniac/eau). On obtient finalement 10,7 g de [2-(1-pipérazinyl)éthoxy]acétamide sous forme d'une huile incolore qui cristallise.
- Rendement:: 57,1 %
- RMN: δ:: 2,33 (4H, m); 2,43 (2H, t, 5,54 Hz); 2,67 (4H, m); 2,79 (1H, s b); 3,53 (2H, t, 5,53 Hz), 3,78 (2H, s); 7,18 (1H, s b); 7,53 (1H, s b).
- Spectre de masse:: 188 (MH⁺).

### I.1.2. 2-(1-pipérazinyl)éthoxyacétonitrile.

Dans un ballon à trois cols de 250 ml équipé d'un condenseur refroidi à l'eau et d'un agitateur mécanique, on introduit 8,6 g (0,1 mole) de pipérazine, 15,9 g (0,1 mole) de dichlorhydrate de pipérazine, 0,6 ml d'eau et 40 ml d'éthanol. On porte la température au mélange à 70°C. On ajoute ensuite goutte à goutte 11,9 g (0,1 mole) de (2-chloroéthoxy)acétonitrile en solution dans 48 ml d'éthanol en l'espace de 15 minutes. On maintient le mélange à 70°C pendant 16 heures. On laisse le mélange revenir à température ambiante puis on le refroidit au bain de glace. On filtre ensuite le précipité qui s'est formé. On concentre le filtrat sous pression réduite à l'évaporateur rotatif et on reprend le résidu (huile + solide) avec 50 ml d'éthanol. On laisse sous agitation pendant 15 minutes. On filtre le précipité qui s'est formé (dichlorohydrate de pipérazine) et le filtrat est concentré sous vide à 50°C à l'évaporateur rotatif.

On purifie le résidu d'évaporation par chromatographie préparative sur gel de silice (éluant: mélange 94,5:5:0,5 (v/v/v): dichlorométhane/méthanol/solution aqueuse d'ammoniac à 28 % (en poids) remplacé progressivement par un mélange 89:10:1 (v/v/v) des mêmes constituants). On obtient ainsi 4,7 g de 2-(1-pipérazinyl)éthoxyacétonitrile sous forme d'une huile orange.
- Rendement:: 27,8 %
- RMN; δ:: 2,36 (4H, m); 2,47 (2H, t, 5,6 Hz); 2,71 (4H, m); 3,6 (2H, t, 5,6 Hz). 4,44 (2H, s).
- Spectre de masse:: 170 (MH⁺).

### I.1.3. 2-(1-pipérazinyl)éthoxyacétate de méthyle.

Dans un ballon à trois cols de 250 ml équipé d'un condenseur refroidi à l'eau et d'un agitateur mécanique, on introduit 8,6 g (0,1 mole) de pipérazine, 17,7 g (0,1 mole) de dichlorhydrate de pipérazine, 10,8 ml (0,6 mole) d'eau et 40 ml de méthanol. On amène la température du mélange à 39°C. On ajoute ensuite goutte-à-goutte 13,8 g (0,1 mole) de l'ester méthylique de l'acide (2-chloroéthoxy)acétique en solution dans 17 ml de méthanol en l'espace de 35 minutes. On maintient le mélange à 65°C pendant 48 heures. On laisse le mélange revenir à la température ambiante et on filtre les sels de pipérazine qui ont précipité. Le filtrat est concentré sous pression réduite à l'évaporateur rotatif à 50°C. On obtient 31,6 g d'une huile jaune que l'on purifie par chromatographie préparative sur gel de silice (éluant: mélange 94,5:5:0,5 (v/v/v) de dichlorométhane/méthanol/solution aqueuse à 28 % (en poids) d'ammoniac remplacé progressivement par un mélange 73,5:25:2,5 (v/v/v) des mêmes constituants). On obtient 9,83 g de 2-(1-pipérazinyl)éthoxyacétate de méthyle sous forme d'une huile incolore.
- rendement:: 48,6 %
- RMN: δ:: 2,54 (2H, t, 5,6 Hz); 2,60 (4H, m); 2,95 (4H, m); 3,58 (2H, t, 5,6 Hz); 3,65 (3H, s); 4,10 (2H, s).
- Spectre de masse:: 202 (M⁺⁻)

### I.1.4. Acide 2-(1-pipérazinyl)éthoxyacétique.

Dans un ballon à trois cols de 100 ml équipé d'un condenseur refroidi à l'eau et d'un agitateur mécanique, on introduit 8,6 g (0,1 mole) de pipérazine, 17,7 g (0,1 mole) de dichlorhydrate de pipérazine et 50 ml d'eau. On porte la température du mélange à 70°C. On ajoute ensuite goutte à goutte 15,2 g d'acide (2-chloroéthoxy)acétique en l'espace de 15 minutes. On porte la température du mélange à 80°C sous agitation et on maintient le mélange à cette température pendant 27 heures. On laisse le mélange revenir à la température ambiante et on évapore l'eau sous pression réduite à l'évaporateur rotatif. Le résidu d'évaporation est repris avec 50 ml d'éthanol et maintenu à 50°C sous agitation pendant 45 minutes. On le place ensuite dans un bain de glace et on agite pendant 1 heure. on filtre ensuite le précipité (dichlorhydrate de pipérazine) qui s'est formé et on évapore les solvants sous pression réduite dans un évaporateur rotatif à 50°C. On obtient 22,4 g d'une huile jaune. On purifie 10 g de ce mélange sur 130 g de résine Amberlyte IRA-400. On élue d'abord avec 600 ml d'eau et ensuite avec une solution aqueuse 0,5 M en acétate d'ammonium. On rassemble les fractions contenant l'acide 2-(1-pipérazinyl)éthoxyacétique ou son sel et on en élimine l'eau sous pression réduite à 60°C à l'évaporateur rotatif. On récupère 18,2 g d'un mélange contenant des cristaux blancs et une huile. On reprend ce mélange avec 75 ml d'isopropanol et les cristaux insolubles sont filtrés. Le filtrat est acidifié avec 20 ml d'une solution d'acide chlorhydrique 9N dans l'éthanol. Le précipité formé est filtré rapidement, lavé à l'isopropanol et séché à l'évaporateur rotatif sous pression réduite à 50°C. On obtient 7,1 g d'un solide blanc que l'on purifie en sublimant les sels de chlorure d'ammonium (4 heures à 135°C sous 0,1 mbar puis 8 heures à 150°C sous 0,1 mbar). On obtient ainsi 1,4 g du dichlorhydrate de l'acide 2-(1-pipérazinyl)éthoxyacétique.
- Rendement:: 12 %.
- RMN: δ:: 2,36 (2H, t, 4,8 Hz); 3,45 (4H, m); 3,53 (4H, m); 3,88 (2H, t, 4,8 Hz); 4,09 (2H, s); 10 (1H, s b).
- Spectre de masse:: 189 (MH⁺)

| Analyse élémentaire: | | | |
|---|---|---|---|
| Atome | C | H | N |
| % Calculé | 36,79 | 6,95 | 10.73 |
| % Trouvé | 36,57 | 7,07 | 10,69 |

### I.2. Préparation de composés de formule I avec R₂ = -CH₂-C₆H₅.

### I.2.1. 2-(4-benzyl-1-pipérazinyl)éthoxyacétamide.

### I.2.1.1.

On introduit 8,8 g (0,05 mole) de 1-benzylpipérazine, 7,6 g (0,055 mole) de (2-chloroéthoxy)acétamide, 11,7 g (0,11 mole) de carbonate de sodium, 0,050 g d'iodure de potassium et 44 ml de méthyléthylcétone dans un ballon à trois cols de 100 ml équipé d'un condenseur refroidi à l'eau et d'un agitateur mécanique. On porte le mélange à la température du reflux et on maintient à cette température pendant 20 heures. On laisse revenir le mélange à la température ambiance et on ajoute 50 ml d'eau. On élimine ensuite la méthyléthylcétone *sous* pression réduite à l'évaporateur rotatif. On extrait ensuite la phase aqueuse avec 2 x 50 ml de dichlorométhane. On rassemble les phases organiques et on les lave avec 25 ml d'une solution saturée en chlorure d'ammonium. On sèche sur sulfate de sodium puis on filtre et on concentre sous pression réduite à l'évaporateur rotatif. On obtient 14,15 g d'une huile brune qui cristallise et que l'on purifie par chromatographie préparative sur gel de silice (éluant: mélange 97:3:0,3 (v/v/v) dichlorométhane/méthanol/solution aqueuse à 28 % d'ammoniac). On obtient 11.5 g d'un solide jaune.
- Rendement:: 82,9 %

Après recristallisation dans l'acétate d'éthyle, on obtient le 2-(4-benzyl-1-pipérazinyl)éthoxyacétamide sous forme d'un solide blanc.
- Rendement de cristallisation:: 81,5 %.
- RMN: δ :: 2,40 (8H, m) ; 2,47 (2H, t, 5,58 Hz); 3,44 (2H, s); 3,53 (2H, t, 5,57 Hz); 3,78 (2H, s); 7,19 (1H, s b); 7,31-7,22 (5H, m); 7,34 (1H, s b).
- Spectre de masse:: 277 (M⁺⁻).
- DSC:: onset 74,6°C, Max: 78,6°C.

| Analyse élémentaire: | | | |
|---|---|---|---|
| Atome | C | H | N |
| % Calculé | 64,95 | 8,36 | 15,15 |
| % Trouvé | 65,12 | 8,70 | 15,10 |

### I.2.1.2.

On introduit dans un ballon à trois cols de 100 ml équipé d'un condenseur refroidi à l'eau et d'un agitateur mécanique, 8,8 g (0,05 mole) de 1-benzylpipérazine, 12,6 g (0,055 mole) de (2-iodoéthoxy)acétamide, 11,7 g (0,11 mole) de carbonate de sodium et 44 ml de méthyléthylcétone. On porte le mélange à la température du reflux et on maintient le mélange à cette température pendant 4 heures. On laisse revenir le mélange à la température ambiante et on ajoute 75 ml d'eau. On élimine ensuite la méthyléthylcétone sous pression réduite à l'évaporateur rotatif. On extrait ensuite la phase aqueuse avec 75 puis 50 ml de dichlorométhane. On rassemble les phases organiques et on les sèche sur sulfate de sodium puis on filtre et on concentre sous pression réduite à l'évaporateur rotatif. On obtient 14,5 g d'une huile orange qui cristallise et que l'on purifie par chromatographie préparative sur gel de silice (éluant: mélange 95,6:4:0,4 (v/v/v) de dichlorométhane/méthanol/solution aqueuse d'ammoniac à 28 % puis mélange 93,6:6:0,4 (v/v/v) des mêmes constituants. On obtient finalement 12,7 g (91,6 %) d'un solide jaune. Après recristallisation dans l'acétate d'éthyle, on obtient le 2-(4-benzyl-1-pipérazinyl)éthoxyacétamide sous forme d'un solide blanc (rendement de cristallisation: 81,5 %).
Analyses: voir exemple I.2.1.1.

### I.2.2. 2-(4-benzyl-1-pipérazinyl)éthoxyacétonitrile,

Dans un ballon à trois cols de 100 ml équipé d'un condenseur refroidi à l'eau et d'un agitateur mécanique, on introduit 8,8 g (0,05 mole) de 1-benzylpipérazine, 6,6 g (0,055 mole) de (2-chloroéthoxy)acétonitrile, 11,5 g (0,11 mole) de carbonate de sodium, 0,5 g d'iodure de potassium et 50 ml de méthyléthylcétone. On porte la température du mélange à 80°C et on maintient pendant 24 heures. On laisse ensuite revenir le mélange à température ambiante puis on dilue le mélange avec 50 ml d'eau et on élimine la méthyléthylcétone à l'évaporateur rotatif sous pression réduite. On extrait la phase aqueuse avec 2 x 50 ml de dichlorométhane et on rassemble les phases organiques. On les sèche sur sulfate de sodium puis on les filtre. On concentre le filtrat à l'évaporateur rotatif sous pression réduite. On obtient 14.6 g d'une huile brune que l'on purifie par chromatographie préparative sur gel de silice (éluant : mélange 97,8:2:0,2 (v/v/v): dichlorométhane/méthanol/solution aqueuse à 28 % d'ammoniac). On obtient 8,4 g (64,9 %) de 2-(4-benzyl-1-pipérazinyl)éthoxyacétonitrile sous forme d'une huile orange.
RMN (dans le chloroforme deutérié):
- δ:: 2,40 (8H, m); 2,50 (2H, t, 5,68 Hz); 3,44 (2H, s); 3,61 (2H, t, 5,67 Hz); 4,44 (2H, s); 7,28 (5H, m).
- Spectre de masse:: 259 (M⁺⁻)

### I.2.3. 2-(4-benzyl-1-pipérazinyl)éthoxyacétate de méthyle.

Dans un ballon à trois cols de 250 ml équipé d'un condenseur refroidi à l'eau et d'un agitateur mécanique, on introduit 10 g (0,057 mole) de 1-benzylpipéraxine, 9,52 g (1,1 ég) de (2-chloroéthoxy)acétate de méthyle, 13,23 g (2,2 éq.) de carbonate de sodium, 0,4 g d'iodure de potassium et 100 ml de toluène. Le mélange est chauffé à 100°C pendant 36 heures. On laisse ensuite revenir le mélange à température ambiante, puis on ajoute 100 ml d'eau et on décante la phase organique. On lave la phase organique avec 100 ml d'eau et 100 ml d'une solution saturée en chlorure de sodium. On sèche sur sulfate de sodium puis on filtre et concentre à l'évaporateur rotatif sous pression réduite. On obtient 18,8 g d'une huile brune que l'on purifie par chromatographie préparative sur gel de silice (éluant : mélange 98,9:1:0,1 (v/v/v) de dichlorométhane/méthanol/solution aqueuse à 28 % d'ammoniac que l'on remplace progressivement par un mélange 91,2:8:0,8 des mêmes composants. On obtient 10,8 g de 2-(4-benzyl-1-pipérazinyl)éthoxyacétate de méthyle sous forme d'une huile orange.
Rendement: 65 %.
- RMM: δ:: 2,38 (8H, m); 2,47 (2H, t, 5,87 Hz); 3,44 (2H, s); 3,56 (2H, t, 5,84 Hz); 3,64 (3H, s); 4,09 (2H, s); 7,28 (5H, m).
- Spectre de masse:: 292 (M⁺⁻)

### I.3. Préparation des composés de formule 1 avec R₂ = -COOCH₂-C₆H₅

I.3.1. 4-(2-carbamoylméthoxyéthyl)-pipérazine-1-carboxylate de benzyle. Dans un ballon à trois cols de 100 ml équipé d'un condenseur refroidi à l'eau et d'un agitateur mécanique, on introduit 6,6 g (0,03 mole) de pipérazine-1-carboxylate de benzyle, 7,6 g (0,033 mole) de (2-iodoéthoxy)acétamide, 7 g (0,066 mole) de carbonate de sodium et 33 ml de méthyléthylcétone. On porte le mélange à la température du reflux et on maintient à cette température pendant 5 heures. On laisse revenir le mélange à la température ambiante, puis on ajoute 75 ml d'eau et on élimine la méthyléthylcétone à l'évaporateur rotatif sous pression réduite. On extrait la phase aqueuse avec 75 puis 50 ml de dichlorométhane puis on rassemble les phases organiques. On les sèche sur sulfate de sodium, puis on filtre et on concentre le filtrat à l'évaporateur rotatif sous pression réduite. On obtient 9,4 g d'un solide beige que l'on purifie par chromatographie préparative sur gel de silice (éluant : mélange 95,4:4:0,4 (v/v/v) dichlorométhane/méthanol/solution aqueuse d'ammoniac à 28 %). On obtient 7,7 g (79,9 %) d'un solide blanc dont on recristallise 7,5 g dans 34 ml d'acétone. On récupère 6,6 g de 4-(2-carbamoylméthoxyéthyl)-pipérazine-1-carboxylate de benzyle sous forme de cristaux blancs (rendement de cristallisation : 88 %).
- RMN: δ:: 2,41 (4H, m); 2,51 (2H, t); 3,39 (4H, m); 3,56 (2H, t, 5,48 Hz); 3,79 (2H, s); 5,08 (2H, s); 7,15 (1H, s él.); 7,35 (6H, m).
- Spectre de masse:: 321 (M⁺⁻).
- DSC:: Onset: 115,09°C, Max: 125,46°C
Onset: 136,04°C, Max: 143,86°C.

| Analyse élémentaire: | | | |
|---|---|---|---|
| Atome | C | H | N |
| % Calculé | 59,79 | 7,21 | 13,07 |
| % Trouvé | 59,97 | 7,47 | 12,98 |

### I.3.2. 4-(2-cyanométhoxyéthyl)-pipérazine-1-carboxylate de benzyle.

Dans un ballon à trois cols de 100 ml équipé d'un condenseur refroidi à l'eau et d'un agitateur mécanique, on introduit 6,4 g (0,029 mole) de piperazine-1-carboxylate de benzyle, 3,8 g (0,0319 mole) de (2-chloroéthoxy)acétonitrile, 6,8 g (0,0638 mole) de carbonate de sodium, 20 mg d'iodure de potassium et 32 ml de méthyléthylcétone. On porte ce mélange à la température du reflux (80°C) pendant 20 heures. On ajoute alors 1,7 g (0,0145 mole) de (2-chloroéthoxy)acétonitrile et on maintient à la température du reflux pendant 24 heures supplémentaires. On laisse revenir à température ambiante, puis on ajoute 75 ml d'eau et élimine la méthyléthylcétone à l'évaporateur rotatif sous pression réduite. On extrait la phase aqueuse avec 75 puis 50 ml de dichlorométhane. On rassemble les phases organiques et on les sèche sur sulfate de sodium. On filtre et le filtrat est concentré à l'évaporateur rotatif sous pression réduite. On obtient 10,5 g d'un liquide brun-foncé que l'on purifie par chromatographie préparative sur gel de silice (éluant : mélange 99:1:0,1 (v/v/v) dichlorométhane/méthanol/solution aqueuse d'ammoniac à 28 % remplacé progressivement par un mélange 98:2:0,1 des mêmes constituants. On obtient 7,3 g (83 %) de 4-(2-cyanométhoxyéthyl)-pipérazine-1-carboxylate de benzyle sous forme d'un liquide jaune.
- RMN: δ:: 2,39 (4H, m); 2,54 (2H, t, 5,5 Hz); 3,39 (4H, m); 3,63 (2H, t, 5,3 Hz); 4,45 (2H, s); 5,07 (2H, s); 7,35 (5H, m).
- Spectre de masse:: 304 (MH⁺).

### I.4. Préparation de composés de formule I avec R₂ = -COOtBu.

### I.4.1. 4-(2-carbamoylméthoxyéthyl)-pipérazine-1-carboxylate de tert-butyle.

Dans un ballon à trois cols de 100 ml équipé d'un condenseur refroidi à l'eau et d'un agitateur mécanique, on introduit 5,6 g (0,03 mole) de piperazine-1-carboxylate de tert-butyle, 7,6 g (0,033 mole) de (2-iodoéthoxy)acétamide, 7 g (0,066 mole) de carbonate de sodium et 28 ml de méthyléthylcétone. On porte le mélange à la température du reflux pendant 2 heures. On laisse revenir à température ambiante, puis on ajoute 75 ml d'eau et on élimine la méthyléthylcétone à l'évaporateur rotatif sous pression réduite. La phase aqueuse est extraite avec 2x50 ml de dichlorométhane. Les phases organiques sont rassemblées, lavées avec 40 ml d'une solution aqueuse saturée en chlorure d'ammonium, séchées sur sulfate de sodium, filtrées et concentrées à l'évaporateur rotatif sous pression réduite. On obtient 9,7 g d'un solide jaune-pâle que l'on purifie par chromatographie prépararive sur gel de silice (éluant : mélange 98:2:0,2 (v/v/v) dichlorométhane/méthanol/solution aqueuse d'ammoniac à 28 %). On obtient 7,8 g (90,5 %) d'un solide blanc dont on recristallise 7,7 g dans 15,4 ml d'acétone. On obtient finalement 6,35 g de 4-(2-carbamoylméthoxyéthyl)-pipérazine-l-carboxylate de tert-butyle sous forme de cristaux blancs (rendement de cristallisation : 82,5 %).
- RMN: δ:: 1,39 (9H, s); 2,38 (4H, m); 2,49 (2H, t); 3,29 (4H, m) ; 3,55 (2H, t, 5,5 Hz); 3,75 (2H, s); 7,14 (1H, s él); 7,38 (1H, s él).
- Spectre de masse:: 287 (M^{+.}).
- DSC:: onset 113,7°C, Max: 117,9°C.

| Analyse élémentaire: | | | |
|---|---|---|---|
| Atome | C | H | N |
| % Calculé | 54,33 | 8,77 | 14,62 |
| % Trouvé | 54,73 | 8,95 | 14,84 |

### I.4.2. 4-(2-cyanométhoxyéthyle)-pipérazine-1-carboxylate de tert-butyle.

Dans un ballon à trois cols de 250 ml équipé d'un condenseur refroidi à l'eau et d'un agitateur mécanique, on introduit 20 g (0,107 mole) de pipérazine-1-carboxylate de tert-butyle, 14,12 g (0,117 mole) de (2-chloroéthoxy)acétonitrile. 25,04 g (0,235 mole) de carbonate de sodium, 0,075 g d'iodure de potassium et 100 ml de méthyléthylcétone. On porte le mélange à la température du reflux pendant 78 heures. On laisse le mélange revenir à 50°C et on filtre les sels. Les solvants sont évaporés à l'évaporateur rotatif sous pression réduite. On reprend le résidu avec 150 ml d'eau et on extrait avec 200 puis 100 ml de dichlorométhane. Les phases organiques sont rassemblées, séchées sur sulfate de sodium, filtrées et concentrées à l'évaporateur rotatif sous pression réduite. On obtient 30,5 g d'une huile brune que l'on purifie par chromatographie préparative sur gel de silice (éluant : dichlorométhane, puis un mélange 98,9:1:0,1 (v/v/v) dichlorométhane/méthanol/solution aqueuse d'ammoniac à 28 % progressivement remplacée par un mélange 97,8:2:0,2 des mêmes constituants. On obtient 25,8 g de 4-(2-cyanométhoxyéthyle)-pipérazine-1-carboxylate de tert-butyle sous forme d'une huile jaune.
Rendement: 89,2 %
RMN dans le chloroforme deutérié:
- δ:: 1,44 (9H, s); 2,43 (4H, m); 2,62 (2H, t, 5,4 Hz); 3,43 (4H, m); 3,71 (2H, t, 5,4 Hz); 4,28 (2H, s).
- Spectre de masse:: 269 (M⁺⁻).

### I.4.3. 4-(2-méthoxycarbonylméthoxyéthyle)-pipérazine-1-carboxylate de tert-butyle.

Dans un ballon à trois cols de 250 ml équipé d'un condenseur refroidi à l'eau et d'un agitateur mécanique, on introduit 7,06 g (0,0379 mole) de pipérazine-1-carboxylate de tert-butyle, 6,35 g (0,0417 mole) de (2-chloroéthoxy)acétate de méthyle, 8,83 g (0,083 mole) de carbonate de sodium, 0,025 g d'iodure de potassium et 80 ml de toluène. On porte le mélange à la température du reflux pendant 78 heures. On laisse le mélange revenir à la température ambiante et filtre les sels. Le filtrat est lavé avec 2x50 ml d'eau. On extrait la phase aqueuse avec 50 ml de dichlorométhane. On rassemble les phases organiques et on les sèche sur sulfate de sodium. On filtre et le filtrat est concentré à l'évaporateur rotatif sous pression réduite. On obtient 12,72 g d'une huile brune que l'on purifie par chromatographie préparative sur gel de silice (éluant : dichlorométhane puis mélange 98,9:1:0,1 (v/v/v) dichlorométhane/méthanol/solution aqueuse d'ammoniac à 28 % puis mélange 97,2:2:0,2 des mêmes constituants. On obtient 7,2 g de 4-(2-méthoxycarbonylméthoxyéthyle)-pipérazine-1-carboxylate de tert-butyle sous forme d'une huile jaune.
Rendement: 62,8 %
RMN (dans le chloroforme deutérié):
- δ:: 1,45 (9H, s); 2,45 (4H, m); 2,63 (2H, t, 5,6 Hz); 3,43 (4H, m); 3,68 (2H, t, 5,6 Hz); 3,74 (3H, s); 4,10 (2H, s).
- Spectre de masse:: 302 (M⁺⁻).

### I.5. Préparation des composés de formule I avec R₂ = -COOEt.

### I.5.1. 4-(2-carbamoylméthoxyéthyl)-pipérazine-1-carboxylate d'éthyle.

### I.5.1.1.

Dans un ballon à trois cols de 21 équipé d'un condenseur refroidi à l'eau et d'un agitateur mécanique, on introduit 164 g (1,04 moles) de pipérazinecarboxylate d'éthyle, 156,9 g (1,14 moles) de (2-chloroéthoxy)acétamide, 241,7 g (2,28 moles) de carbonate de sodium, 1 g d'iodure de potassium et 200 ml de toluène. On porte le mélange à la température du reflux pendant 3 heures 30. On laisse le mélange revenir à 50°C, et on ajoute 200 ml d'isopropanol. On filtre le mélange réactionnel et lave le résidu de filtration avec 150 ml d'isopropanol. Le filtrat est concentré à l'évaporateur rotatif sous pression réduite. On obtient 277,2 g de produit brut que l'on recristallise dans 500 ml d'acétate d'éthyle. On obtient 219,2 g (81,6 %) de 4-(2-carbamoylméthoxyéthyl)-pipérazine-1-carboxylate d'éthyle sous forme d'un solide blanc.
- RMN: δ:: 1,24 (3H, t, 7,05 Hz): 2,46 (4H, m); 2,57 (2H, t, 5,2 Hz); 3,44 (4H, m); 3,62 (2H, t, 5,2 Hz); 3,95 (2H, s); 4,1 (2H, q, 7,1 Hz) ; 5,72 (1H, s b); 7,34 (1H, s b).
- Spectre de masse:: 260 (MH⁺).
- DSC:: onset 106,5°C, Max: 109,7°C.

| Analyse élémentaire: | | | |
|---|---|---|---|
| Atome | C | H | N |
| % Calculé | 50,95 | 8,16 | 16,20 |
| % Trouvé | 50,81 | 8,53 | 16,09 |

### I.5.1.2.

Dans un ballon équipé d'un condenseur refroidi à l'eau et d'un agitateur mécanique, on introduit 15,13 g de (2-chloroéthoxy)acétamide (0,11 mole), 14,6 ml de pipérazinecarboxylate d'éthyle (0,1 mole), 23.3 g de carbonate de sodium (0,22 mole) et 1,0 g d'iodure de potassium dans 25 ml de toluène. On chauffe le mélange à la température du reflux pendant 4 heures et on laisse revenir à la température ambiante. On laisse encore la réaction se poursuivre, toujours sous agitation, pendant 16 heures. On ajoute 100 ml d'isopropanol et on filtre les matières solides qui se sont formées. On évapore le solvant du filtrat sous pression réduite jusqu'à siccité. Le produit obtenu après évaporation des solvants est recristallisé dans le toluène. On obtient 21,35 g de 4-(2-carbamoylméthoxyéthyl)-pipérazine-1-carboxylate d'éthyle. Rendement: 82 %.
Spectre de masse: 260 (MH⁺), 214 (M - OEt).

### I.5.2. 2-(4-carboxyéthyl-1-pipérazinyl)éthoxyacétate de potassium.

Dans un ballon à trois cols de 2 1 équipé d'un condenseur refroidi à l'eau et d'un agitateur mécanique, on introduit 200 g (0,77 moles) de 4-(2-carbamoylméthoxyéthyl)-pipérazine-l-carboxylate d'éthyle préparé à l'exemple I.5.1., 216,4 g (3,86 moles) d'hydroxyde de potassium, et 800 ml d'éthanol. On porte le mélange à la température du reflux pendant 24 heures. On laisse le mélange revenir à température ambiante et on filtre les sels. On concentre le filtrat à l'évaporateur rotatif sous pression réduite et l'huile obtenue est reprise par 300 ml d'isopropanol. Les sels sont filtrés sur dicalite et la solution est de nouveau concentrée à l'évaporateur rotatif sous pression réduite. L'huile est reprise par 1,25 1 d'acétate d'éthyle sous agitation. Un précipité apparaît. Le mélange est refroidi au bain de glace pendant 2 heures puis filtré. Le solide blanc obtenu est séché à l'étuve. On obtient 231,8 g de produit contenant des sels inorganiques.

### Exemple II. Déprotection des [2-(1-pipérazinyl)éthoxy]méthyle substitués de formule I dans laquelle R₂ est autre qu'un atome d'hydrogène.

### II.2. 2-(1-pipérazinyl)éthoxyacétamide.

II.2.1. Dans un ballon à trois cols de 250 ml équipé d'un condenseur refroidi à l'eau et d'un agitateur mécanique, on introduit 13,9 g (0,05 mole) de 2-(4-benzyl-1-pipérazinyl)éthoxyacétamide préparé à l'exemple I.2.1. et 139 ml d'éthanol. On ajoute ensuite 1,4 g de palladium sur carbone (10 % en poids) et 15,8 g de formiate d'ammonium. On chauffe le mélange à 30°C pendant 30 minutes, ensuite à 40°C pendant 1 heure et encore à 60°C pendant 30 minutes. On laisse le mélange revenir à 40°C et on filtre sur terres de diatomées (dicalite). On lave le palladium avec de l'éthanol. On concentre le filtrat à l'évaporateur rotatif sous pression réduite. On obtient 9,5 g (100 %) de 2-(1-pipérazinyl)éthoxyacétamide sous forme d'une huile incolore qui cristallise.
Les analyses correspondent à celles obtenues avec le composé préparé à 5 l'exemple I.1.1.

### II.3. A partir des composés de formule I avec R=-COOCH₂-C₆H₅.

### II.3.1. 2-(1-pipérazinyl)éthoxyacétamide.

On introduit dans une bombe de Parr 6,45 g (0,02 mole) de 4-(2-carbamoylméthoxyéthyl)-pipérazine-1-carboxylate de benzyle préparé à l'exemple I.3.1., 0,654 g de palladium sur carbone et 65 ml d'éthanol. On agite le mélange à température ambiante sous une pression de 310,26 kPa pendant 4 heures. On filtre sur dicalite, lave à l'éthanol et concentre le filtrat à l'évaporateur rotatif sous pression réduite. On récupère 3,75 g (100 %) de 2-(1-pipérazinyl)éthoxyacétamide sous forme d'une huile incolore qui cristallise.

Les analyses correspondent à celles obtenues avec le composé préparé à l'exemple I.1.1.

### II.4. A partir des composés de formule I avec R=-COOtBu.

### II.4.1. 2-(1-pipérazinyl)éthoxyacétamide.

Dans un ballon à trois cols de 50 ml équipé d'un condenseur refroidi à l'eau et d'un agitateur mécanique, on introduit 1,4 g (0,005 mole) de 4-(2-carbamoylméthoxyéthyl)-pipérazine-1-carboxylate de tert-butyle préparé à l'exemple I.4.1. et 14 ml d'une solution 3M d'acide chlorhydrique dans l'acétate d'éthyle. On agite le mélange à température ambiance pendant 2 heures. On filtre le précipité et le lave à l'acétate d'éthyle. On obtient 1,3 g (100 %) du dichlorhydrate de la 2-(1-pipérazinyl)éthoxyacétamide.
- RMN: δ:: 3,4 (2H, t, 4,7 Hz); 3,49-3,66 (8H, m); 3,81 (2H, t, 4,7 Hz); 3,87 (2H, s); 6,2 (5H échangeables, m b); 7,2-7,7; 10,2.
- Spectre de masse:: 188 (MH⁺).

### II.4.2. 2-(1-pipérazinyl)éthoxyacétonitrile.

Dans un ballon à trois cols de 250 ml équipé d'un condenseur refroidi à l'eau, d'une ampoule à addition et d'un agitateur mécanique, on solubilise 2,7 g (0,010 mole) de 4-(2-cyanométhoxyéthyle)-pipérazine-l-carboxylate de tert-butyle préparé à l'exemple I.4.2. dans 70 ml de dichlorométhane. On ajoute 1,7 ml (1,2 éq.) d'iodure de triméthylsilyle en solution dans 15 ml de dichlorométhane en 30 minutes. En fin d'addition, on observe la formation d'un précipité. On ajoute 15 ml de dichlorométhane au mélange réactionnel.

Après 20 minutes à température ambiante, on ajoute 1 ml d'iodure de triméthylsilyle en solution dans 10 ml de dichlorométhane en 10 minutes. On agite le mélange à température ambiante pendant 1 heure supplémentaire et laisse reposer pendant 16 heures à température ambiante. On ajoute alors 20 ml de méthanol et agite pendant 10 minutes. Les solvants sont évaporés à l'évaporateur rotatif sous pression réduite à 50°C pour donner 3,5 g d'un solide brun que l'on reprend dans 40 ml de dichlorométhane. On agite le mélange 10 minutes à 35°C. Le précipité qui s'est formé est filtré, rincé avec 2 x 5 ml de dichlorométhane et séché. On obtient 1,6 g (94,6 %) du diiodhydrate de 2-(1-pipérazinyl)éthoxyacétonitrile sous forme d'un solide jaune.
- RMN: δ:: 3,88 (10H, m); 3,87 (2H, t, 4,9 Hz); 4,57 (2H, s); 8,8 (2H, s b).
- Spectre de masse:: 169 (M⁺⁻).

| Analyse élémentaire: | | | |
|---|---|---|---|
| Atome | C | H | N |
| % Calculé | 22,61 | 4,03 | 9,89 |
| % Trouvé | 22,84 | 4,14 | 9,88 |

### II.4.3. 2-(1-pipérazinyl)éthoxyacétate de méthyle.

Dans un ballon à trois cols de 50 ml équipé d'un condenseur refroidi à l'eau et d'un agitateur mécanique, on dissout 2 g (0,0066 mole) de 4-(2-méthoxycarbonylméthoxyéthyle)-pipérazine-1-carboxylate de tert-butyle préparé à l'exemple I.4.3. dans 5 ml d'acétate d'éthyle. On ajoute en une fois sous agitation 20 ml d'une solution 3M d'acide chlorhydrique dans l'acétate d'éthyle. On agite le mélange à température ambiante pendant 30 minutes. On ajoute alors 5 ml d'une solution 3M d'acide chlorhydrique dans l'acétate d'éthyle et poursuit l'agitation pendant 30 minutes. On filtre le précipité et le lave avec 2 x 5 ml d'acétate d'éthyle. On obtient 1,68 g (92 %) du dichlorhydrate du 2-(1-pipérazinyl)éthoxyacétate de méthyle sous forme d'un solide beige.
- RMN: δ:: 3,4 (2H, t, 4,8 Hz), 3,47-3,65 (8H, m); 3,67 (3H, s); 3,92 (2H, t, 4,8 Hz); 4,21 (2H, s); 10,1 (2H, s b).
- Spectre de masse:: 202 (M⁺⁻).

### II.4.4. 2-(1-pipérazinyl)éthoxyacétate d'éthyle.

Dans un ballon à trois cols de 50 ml équipé d'un condenseur à eau et d'un agitateur mécanique, on solubilise 2 g (0,0066 mole) de 4-(2-méthoxycarbonylméthoxyéthyle)-pipérazine-1-carboxylate de tert-butyle préparé à l'exemple I.4.3. dans 10 ml d'éthanol. On ajoute en une fois sous agitation 11 ml d'une solution 3,8M d'acide chlorhydrique dans l'éthanol. On agite le mélange à température ambiante pendant 30 minutes et on ajoute encore 11 ml d'une solution 3,8M d'acide chlorhydrique dans l'éthanol. On porte le mélange à la température du reflux pendant 4 heures. On élimine le solvant à l'évaporateur rotatif sous pression réduite. On obtient 1,86 g (97,3 %) du dichlorhydrate du 2-(1-pipérazinyl)éthoxyacétate d'éthyle sous forme d'une huile jaune-pâle qui cristallise.
- RMN: δ:: 1,21 (3H, t, 7,1 Hz); 3,37 (2H, t, 4,8 Hz); 3,46 (4H, m); 3,55 (4H, m); 3,91 (2H, t, 4,8 Hz); 4,14 (2H, q, 7,1 Hz); 4,18 (2H, s); 10,1 (1H, s b); 12 (1H, s b).
- Spectre de masse:: 216 (M⁺⁻).

### II.5. A partir de composés de formule I avec R₂ = -COOEt.

### II.5.1. acide 2- (1-pipérazinyl)éthoxyacétique.

Dans un ballon muni d'un agitateur mécanique et d'un condenseur refroidi à l'eau, on met en suspension 20 g (0,077 mole) de 4-'(2-carbamoylméthoxyéthyl)-pipérazine-1-carboxylate d'éthyle préparé à l'exemple 1.2 dans un mélange de 20 ml d'une solution aqueuse à 37 % en poids d'acide chlorhydrique et de 20 ml d'eau, et, sous agitation, on porte la température du mélange à 50°C.

On laisse réagir à cette température pendant 5 heures. On refroidit le mélange réactionnel jusqu'à 0°C et on ajuste le pH du mélange à 6 au moyen d'une solution aqueuse d'hydroxyde de sodium à 50 %. On évapore l'eau du milieu réactionnel d'abord simplement sous pression réduite puis, toujours sous pression réduite, après avoir ajouté du toluène. On évapore ensuite le toluène au moyen d'un évaporateur rotatif puis on reprend le résidu d'évaporation dans 100 ml d'isopropanol. On filtre les sels qui se sont formés et on acidifie le filtrat par addition de 28 ml d'une solution aqueuse 6N d'acide chlorhydrique. On évapore l'eau du mélange d'abord simplement sous pression réduite puis, toujours sous pression réduite, après avoir ajouté 50 ml de toluène. On évapore ensuite le toluène au moyen d'un évaporateur rotatif puis on reprend le résidu d'évaporation dans 50 ml d'acétone. On filtre les cristaux blancs qui se sont formés et on évapore l'acétone du filtrat sous pression réduite. On obtient ainsi une huile qui est engagée telle quelle dans l'étape suivante.

On dissout l'huile ainsi obtenue dans 75 ml d'une solution 5,5 N d'hydroxyde de potassium dans l'éthanol et on porte à la température du reflux pendant 28 heures. On ajoute 100 ml d'eau et on évapore l'éthanol sous pression réduite, puis on ajuste le pH du mélange à 7 avec 36 ml d'une solution aqueuse 6N d'acide chlorhydrique. Après avoir éliminé l'eau par évaporation sous pression réduite, on reprend le résidu d'évaporation dans l'isopropanol et on filtre les matières solides. On évapore le solvant du filtrat et on dissout l'huile résiduelle ainsi obtenue dans 30 ml d'une solution aqueuse d'acide chlorhydrique 6N. On élimine à nouveau l'eau par évaporation sous pression réduite et on reprend le solide avec 100 ml de toluène. On le filtre et on le lave avec du toluène. On obtient 9,4 g du dichlorhydrate de l'acide 2-(1-pipérazinyl)éthoxyacétique. Rendement (65 %)
Spectre de masse: 189 (MH⁺), 171 (M-OH), 99 (HN(C₄H₈)N⁺=CH₂).

### Exemple III. Conversion du groupe R₁ des composés de formule I en un groupe acide carboxylique.

### III.1. Acide 2-(4-benzyl-1-pipérazinyl)éthoxyacétique.

III.1.A. Dans un ballon à trois cols de 100 ml équipé d'un condenseur refroidi à l'eau et d'un agitateur magnétique, on dissout 13,9 g (0,05 mole) de 2-(4-benzyl-1-piperazinyl)éthoxyacétamide dans 15 ml d'eau. On ajoute 31 ml (0,375 mole) d'une solution aqueuse d'acide chlorhydrique à 37 %. On chauffe le mélange à 60°C pendant 1 heure. On élimine l'eau à l'évaporateur rotatif sous pression réduite à 60°C. On reprend le solide blanc obtenu dans 75 ml d'acétone et on agite pendant 1 heure à température ambiante. On filtre et obtient 19,7 g du dichlorhydrate d'acide 2-(4-benzyl-1-pipérazinyl)éthoxyacétique sous forme de cristaux blancs.

### Purification :

1) Recristallisation : 5 g de cristaux blancs sont solubilisés à chaud dans 55 ml d'un mélange 9:1 (v/v) isopropanol/eau. On récupère 2 g de 2-(4-benzyl-1-pipérazinyl)éthoxyacétique produit soit 44,8 % de rendement.
2) Sublimation : 2 g de cristaux blancs finement broyés sont placés dans un sublimateur Büchi. L'appareil est mis sous vide (0,2 mmHg) et chauffé à 150°C durant 11 heures. On récupère 1,65 g de 2-(4-benzyl-1-pipérazinyl)éthoxyacétique pur soit 92,8 % de rendement.

- RMN: δ:: 3,39 (6H, m) ; 3,65 (4H, m); 3,88 (2H, t, 4,6 Hz): 4,09 (2H, s); 4,34 (2H, s d); 7,44 (3H, m); 7,64 (2H, m).
- Spectre de masse:: 278 (M^{+.}).

### Obtention de la base libre :

On dissout 5 g de cristaux blancs dans 25 ml d'eau. Le pH de la solution est amené à 7 par addition de 25 ml d'une solution aqueuse 1N d'hydroxyde de sodium. On élimine l'eau à l'évaporateur rotatif sous pression réduite à 60°C et le résidu d'évaporation est repris dans 50 ml d'isopropanol. On agite le mélange à l'évaporateur rotatif à 50°C pendant 2 heures. On filtre les sels et le filtrat est concentré à l'évaporateur rotatif sous vide à 50°C. On obtient 4 g d'une huile orange. Cette huile est reprise dans 40 ml d'acétone et la solution est agitée à 50°C. Les sels sont filtrés et après concentration du filtrat, on obtient 3,4 g (96,3 %) de l'acide 2-(4-benzyl-1-pipérazinyl)éthoxyacétique sous forme d'une huile brune.
- RMN: δ:: 2,51 (4H, m); 2,78 (6H, m); 3,54 (2H, s); 3,66 (2H, t, 5,4 Hz); 3,92 (2H, s); 7,28 (5H, m).
- Spectre de masse:: 279 (MH⁺).
- DSC:: onset 234,05°C, Max: 237,7°C.

| Analyse élémentaire: | | | |
|---|---|---|---|
| Atome | C | H | N |
| % Calculé | 51,29 | 6,89 | 7,98 |
| % Trouvé | 51,38 | 7,16 | 7,94 |

### III.1.B. Acide 2-(1-pipérazinyl)éthoxyacétique).

On place dans une bombe de Parr 2 g (0,0057 mole) du dichlorhydrate de l'acide 2-(4-benzyl-1-piperazinyl)éthoxyacétique, 0,2 g de palladium sur carbone et 50 ml d'une solution 8:2 (v/v) éthanol/eau. On agite le mélange à température ambiante sous une pression de 310,26 kPa pendant 5 heures. On filtre sur dicalite, lave avec 25 ml d'une solution 8:2 (v/v) éthanol/eau et on concentre le filtrat à l'évaporateur rotatif sous pression réduite. On récupère 1,6 g d'un solide orange. Ce solide est repris dans 10 ml d'éthanol et agité pendant 1 heure. Après filtration et séchage, on récupère 1,2 g du dichlorhydrate de l'acide 2-(1-pipérazinyl)éthoxyacétique) sous forme de cristaux jaunes (80,6 %).

Les analyses correspondent à celles obtenues avec le composé préparé à l'exemple I.1.4.

### III.2. Hydrolyse et déprotection en 1 étape.

Dans un erlenmeyer de 10 ml, on place 1 g (0,00348 mole) de 4-(2-carbamoylméthoxyéthyl)pipérazine-1-carboxylate de tert-butyle préparé à l'exemple I.4.3. et 2 ml d'eau. On ajoute 2 ml d'une solution d'acide chlorhydrique à 37 % et on chauffe au bain d'huile à 60°C pendant 1 heure. L'eau est éliminée à l'évaporateur rotatif sous pression réduite. Le résidu est repris au toluène. Après évaporation, on obtient 1,1 g d'un solide jaune pâle. Ce solide finement broyé est placé dans un sublimateur Büchi. L'appareil est placé sous vide (0,3 mmHg) et chauffé à 150°C pendant 8 heures. On obtient 800 mg (88 %) de dichlorhydrate de l'acide 2-(1-pipérazinyl)éthoxyacétique sous forme de cristaux blancs.

Les analyses correspondent à celles obtenues avec le composé préparé à l'exemple I.1.4.

### Exemple IV. Préparation des composés de formule II.

### IV.1. Au départ du chlorure de (4-chlorophényl)phénylméthane.

IV.1.1. 2-[(4-[(4-chlorophényl)phénylméthyl)-1-pipérazinyl]éthoxy]acétamide-Dans un ballon à trois cols de 100 ml équipé d'un condenseur refroidi à l'eau et d'un agitateur mécanique, on introduit 2,4 g (0,01 mole) de chlorure de (4-chlorophényl)phénylméthane, 4,1 g (0,022 mole) de 2-(1-pipérazinyl)éthoxyacétamide préparé à l'exemple I.1.1. et 10 ml d'acétonitrile. On porte le mélange à reflux pendant 3 heures. on laisse le mélange revenir à température ambiante et on concentre le mélange sous pression réduite à l'évaporateur rotatif. Le résidu est repris avec 25 ml d'eau. Le pH de la solution est amené à 14 avec une solution NaOH 1N. On extrait ensuite avec 2 x 25 ml de dichlorométhane. Les phases organiques sont rassemblées, lavées avec une solution saturée en chlorure de sodium, séchées sur sulfate de sodium, filtrées et concentrées à l'évaporateur rotatif sous pression réduite à 50°C. On obcient 4,1 g d'une huile brune que l'on purifie par chromatographie préparative sur gel de silice (éluant : mélange 98:2:0,2 (v/v/v) dichlorométhane/méthanol/solution aqueuse d'ammoniac à 28 % puis par un mélange 97:3 (v/v) dichlorométhane/méthanol). On obtient donne 3,2 g (82,5 %) de 2-[(4-[(4-chlorophényl)phénylméthyl)-1-pipérazinyl]éthoxy]acétamide sous forme d'une huile incolore qui cristallise.

### IV.1.2. Acide 2-[2-[4-[(4-chlorophényl)phénylméthyl]-1-pipérazinyl]éthoxy]acétique.

Dans un ballon à trois cols de 100 ml équipé d'un condenseur refroidi à l'eau et d'un agitateur mécanique, on introduit 5,9 g (0,025 mole) de chlorure de (4-chlorophényl)phénylméthane, 11,3 g (0,05 mole) de 2-(1-pipérazinyl)éthoxyacétate de potassium et 50 ml d'acétonitrile. On porte le mélange à reflux pendant 16 heures. On laisse le mélange revenir à température ambiance, on décante l'acétonitrile et reprend le solide brun dans 50 ml d'eau. La phase aqueuse et la phase acétonitrile sont rassemblées et concentrées à l'évaporateur rotatif sous pression réduite. Le résidu (solide brun) est repris par 50 ml d'eau et le pH de la solution est amené à 4-5 avec 8 ml d'une solution aqueuse d'acide chlorhydrique 6N. On extrait ensuite avec 2 x 50 ml de dichlorométhane. Les phases organiques sont rassemblées, séchées sur sulfate de sodium, filtrées et concentrées sous vide à 50°C. On obtient 9,5 g d'une huile brune. Cette huile est solubilisée dans 100 ml d'acétone. On ajoute 2 g de Norit et agite 15 minutes à 50°C. Après filtration, on obtient une solution orange. On ajoute 4,6 ml d'HCl concentré (37 %). L'acétone est évaporée à l'évaporateur rotatif sous pression réduite et le résidu huileux brun est repris par 100 ml d'acétone. On observe la formation d'un précipité. La suspension est agitée 30 minutes à 50°C puis 2 heures à température ambiante. Après filtration et séchage à 50°C, on obtient 5,6 g (48,5 %) de 2-[2-[4-((4-chlorophényl)phénylméthyl]-1-pipérazinyl]éthoxy]acétique sous forme d'un solide blanc.

### IV.2. Au départ du chlorure de bis(4-fluorophényl)méthane.

IV.2.1. (2-[4-[bis(4-fluorophényl)méthyl]-1-pipérazinyl]éthoxy)acétamide. Dans un ballon à trois cols de 50 ml équipé d'un condenseur refroidi à l'eau et d'un agitateur magnétique, on introduit 2,43 g (0,0102 mole) de chlorure de bis(4-fluorophényl)méthane, 3,84 g (0,022 mole) de 2-(1-pipérazinyl)éthoxyacétamide et 10 ml d'acétonitrile. On porte le mélange à reflux pendant 3 heures. On laisse le mélange revenir à température ambiante. Le mélange est concentré sous vide et le résidu est repris avec 100 ml d'eau et 100 ml de dichlorométhane. Le pH de la phase aqueuse est amené à 14 avec une solution NaOH 1N. On décante la phase organique et réextrait ensuite avec 50 ml de dichlorométhane. Les phases organiques sont rassemblées, lavées avec une solution saturée en chlorure de sodium, séchées sur sulfate de magnésium, filtrées et concentrées à l'évaporateur rotatif à 50°C. On obtient 4,02 g d'une huile jaune que l'on purifie par chromatographie préparative sur gel. de silice (éluant : mélange 98,9:1:0,1 (v/v/v) dichlorométhane/méthanol/solution aqueuse d'ammoniac à 28 % remplacé progressivement par un mélange 95,6:4:0,4 (v/v/v) des mêmes constituants). On obtient 2,5 g (63,1 %) de (2-[4-[bis(4-fluorophényl)méthyl]-1-pipérazinyl]éthoxy)acétamide sous forme d'une huile orange.

### IV.2.2. Acide (2-[4-[bis (4-fluorophényl)méthyl]-1-pipérazinyl]éthoxy]acétique.

Dans un ballon à trois cols de 250 ml équipé d'un condenseur refroidi à l'eau et d'un agitateur mécanique, on introduit 6 g (0,025 mole) de chlorure de bis(4-fluorophényl)méthane, 17 g (0,075 mole) de 2-(1-pipérazinyl)éthoxyacétate de potassium et 100 ml d'acétonitrile. On porte le mélange à reflux pendant 10 heures, puis on laisse le mélange revenir à température ambiante- Le mélange est concentré sous pression réduite à l'évaporateur rotatif et le résidu est repris avec 100 ml d'eau. Le pH de la phase aqueuse est amené à 4-5 avec 5 ml d'une solution HCl 6N. On extrait avec 2 x 50 mi de dichlorométhane. Les phases organiques sont rassemblées et concentrées sous vide à 50°C. L'huile obtenue est reprise par 50 ml d'acétone et agitée pendant 30 minutes à température ambiance. L'insoluble est filtré et le filtrat est concentré à l'évaporateur rotatif sous pression réduite. On obtient 8,5 g d'une huile brune que l'on purifie par chromatographie préparative sur gel de silice (éluant : mélange 89:10:1 (v/v/v) de dichlorométhane/méthanol/solution aqueuse d'ammoniac à 28 % remplacé progressivement par un mélange 78:20:2 (v/v/v) des mêmes constituants. On obtient 3,4 g (34,8 %) de 2-[4-[bis(4-fluorophényl)méthyl]-1-pipérazinyl]éthoxy]acétique sous forme d'une huile incolore.

### IV.3. Au départ du bromure de (4-chlorophényl)phénylméthane.

IV.3.1. (2-[4-[bis(4-fluorophényl)méthyl]-1-pipérazinyl]éthoxy)acétamide. Dans un ballon à trois cols de 50 ml équipé d'un condenseur à eau et d'un agitateur magnétique, on introduit 2,85 g (0,01 mole) de bromure de (4-chlorophényl)phénylméthane, 4,1 g (0,022 mole) de 2-(1-pipérazinyl)éthoxyacétamide et 10 ml d'acétonitrile. On porte le mélange à reflux pendant 2 heures. On laisse le mélange revenir à température ambiante. Le mélange est concentré à l'évaporateur rotatif sous pression réduite et le résidu est repris avec 25 ml d'eau et 50 ml de dichlorométhane. Le pH de la phase aqueuse est amené à 14 avec une solution NaOH 1N. On décante la phase organique et réextrait ensuite avec 50 ml de' dichlorométhane. Les phases organiques sont rassemblées, lavées avec une solution saturée en chlorure de sodium, séchées sur sulfate de magnésium, filtrées et concentrées à l'évaporateur rotatif à 50°C. On obtient 4,14 g d'une huile jaune que l'on purifie par chromatographie préparative sur gel de silice (éluant : mélange 98,9:1:0,1 (v/v/v) dichlorométhane/méthanol/solution aqueuse d'ammoniac à 28 % progressivement remplacé par un mélange 95,6:4:0,4 (v/v/v) des mêmes constituants. On obtient 3,4 g (86,5 %) de (2-[4-[bis(4-fluorophényl)méthyl]-1-pipérazinyl)éthoxy)acétamide sous forme d'une huile incolore qui cristallise.

## Revendications

1. [2-(1-pipérazinyl)éthoxy]méthyle substitués de formule 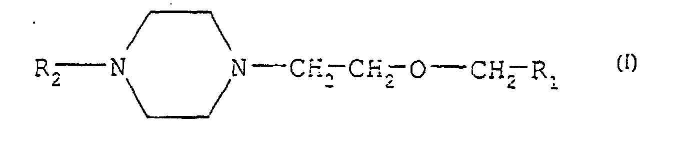 dans laquelle
R₁ représente un groups -CONH₂, -CN, -COOH, -CCOM ou -COOR₃, M étant un métal alcalin et R₃ étant un radical alkyl ayant de 1 à 4 atomes de carbones; et
R₂ représente un atome d'hydrogène ou un groupe -COR₄ ou -R₅, ou R₄ est choisi parmi les groupes -OR₆ ou -R₇ dans lesquels
R₅ représente un radical allyle ou alkylaryle,
R₆ représente un radical alkyle linéaire ou ramifié ayant de 1 à 4 atomes de carbone, un radical halogénoalkyle, alkylaryle, alkylnitroaryle ou alkylhalogénoaryle, et
R₇ représente un halogénoalkyle.

2. [2-(1-pipératinyl)éthoxy)méthyle substitué selon la revendication 1, **caractérisé en ce qu'**il est choisi parmi
l'acide 2-(1-pipérazinyl)éthoxyacétique,
le 2-[2-(1-pipérazinyl)éthoxy]acétamide,
le 2-(1-pipérazinyl)éthoxyacétonitrile,
le 2-(1-pipérazinyl)éthoxyacétate de méthyle,
le 2-(1-pipérazinyl)éthoxyacétate d'éthyle,
le 2-(4-benzyl-1-pipérazinyl)éthoxyacétamide,
le 2-(4-benzyl-1-pipérazinyl)éthoxyacétonitrile,
le 2-(4-benzyl-1-pipérazinyl)éthoxyacétate de méthyle,
le 4-(2-carbamoylméthoxyéthyl)-pipératine-1-carboxylate de benzyle,
le 4-(2-cyanométhoxyéthyl)-pipérazine-1-carboxylate de benzyle,
le 4-(2-carbamoylméthoxyéthyl)-pipérazine-1-carboxylate de tert-butyle,
le 4-(2-cyanométhoxyéthyl)-pipérazine-1-carboxylate de tert-butyle,
le 4-(2-méthoxycarbonylméthoxyéthyl)-pipérazine-1-carboxylate de tert-butyle,
le 4-(2-carbamoylméthoxyéthyl)-pipérazine-1-carboxylate d'éthyle, et
le 2-(4-carboxyéthyl-1-pipérazinyl)éthoxyacétate de potassium.

3. [2-(1-pipérazinyl)éthoxy]méthyle substitués selon la revendication 1, **caractérisé en ce que** le groupement protecteur de la fonction amine R₂ est un groupement carboxylate d'alkyle linéaire ou ramifié ayant de 1 à 4 atomes de carbone ou un groupement alkylaryle.

4. Procédé de préparation de [2-(1-pipérazinyl)éthoxy]méthyle substitués selon l'une quelconque des revendications 1 à 3,
**caractérisé en ce que** l'on fait réagir une pipérazine de formule 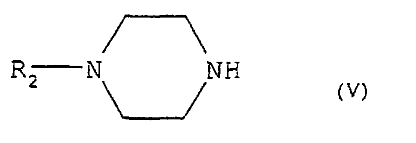 dans laquelle R₂ représente un atome d'hydrogène ou un groupe -COR₄ ou -R₅, où R₄ est choisi parmi les groupes -OR₆ ou -R₇, dans lesquels
R₅ représente un radical allyle ou alkylaryle,
R₆ représente un radical alkyle linéaire ou ramifié ayant de 1 à 4 atomes de carbone, un radical halogénoalkyle, alkylaryle, alkylnitroaryle ou alkylhalogénoaryle, et
R₇ représente un halogéncalkyle,
avec un [2-halogénoéthoxy]méthyle substitué de formule 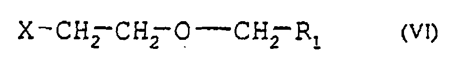 dans laquelle R₁ représente un groupe -CONH₂, -CN, -COOH, -COOM ou -COOR₃, M étant un métal alcalin et R₃ étant un radical alkyle ayant de 1 à 4 atomes de carbone et X représente un atome d'halogène.

5. Procédé selon la revendication 4, **caractérisé en ce que** l'atome d'halogène représenté par X est un atome de chlore ou d'iode.

6. Procédé selon l'une quelconque des revendications 4 ou 5, **caractérisé en ce que** le groupement protecteur de la fonction amine R₂ est un groupement carboxylate d'alkyle linéaire ou ramifié ayant de 1 à 4 atomes ce carbone ou un groupement alkylaryle.

7. Utilisation de [2-(1-pipérazinyl)éthoxy]méthyle substitués de formule 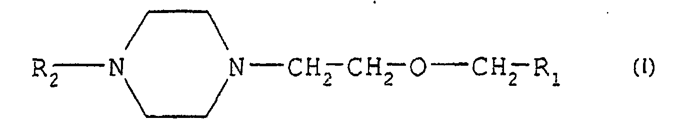 dans laquelle
R₁ représente un groupe -CONH₂, -CN, -COOH, -COOM ou -COOR₃, M étant un métal alcalin et R₃ étant un radical alkyl ayant de 1 à 4 atomes de carbones; et
R₂ représente un atome d'hydrogène,
pour la préparation de composés de formule 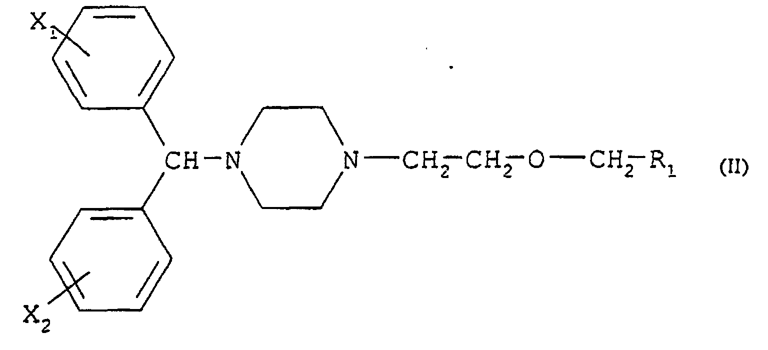 dans laquelle R₁ à la même signification que ci-avant dans la formule (I) et X₁ et X₂ représentent indépendamment un atome d'hydrogène, de fluor, de chlore et/ou de brome, par réaction avec un halogénure de diphénylméthyle de formule 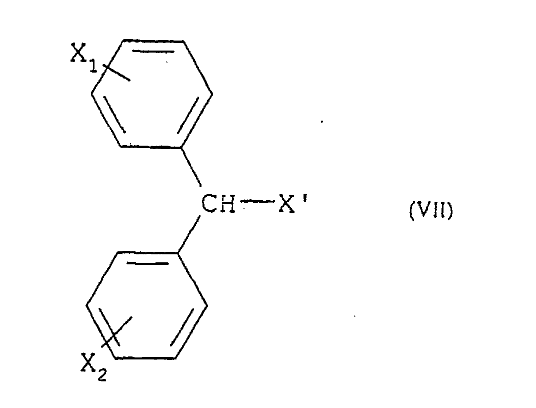 dans laquelle X' représente un atome d'halogène choisi parmi le chlore, le brome ou l'iode et X₁ et X₂ ont la même signification que ci-avant dans la formule (II).

8. Procédé de préparation de composés de formule 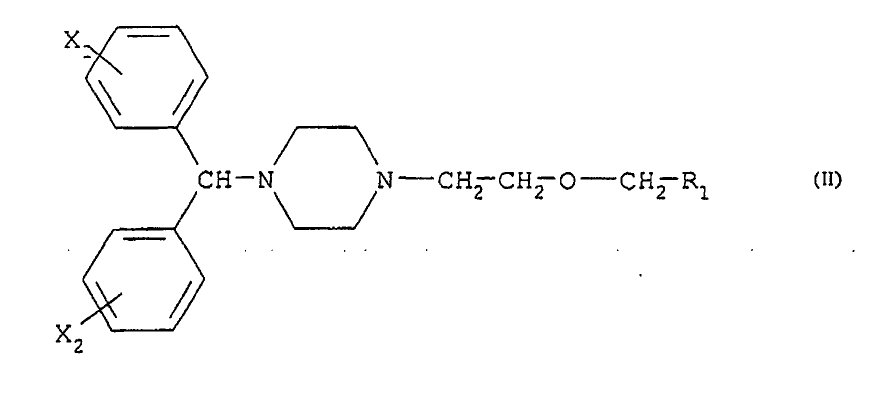 dans laquelle R₁ représente un groupe -CONH₂, -CN, -COOH, -COOM ou -COOR₃, M étant un métal alcalin et R₃ étant un radical alkyl ayant de 1 à 4 atomes de carbones, et X₁ et X₂ représentent indépendamment un atome d'hydrogène, de fluor, de chlore et/ou de brome, **caractérisé en ce que** l'on fait réagir un [2-(1-pipérazinyl)éthoxy]méthyle substitués de formule 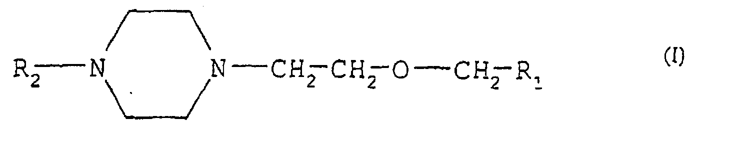 dans laquelle
R₁ représente un groupe -CONH₂, -CN, -COOH, -COOM ou -COOR₃, M étant un métal alcalin et R₃ étant un radical alkyl ayant de 1 à 4 atomes de carbones,
R₂ représente un atome d'hydrogène,
avec un halogénure de diphénylméthyle de formule 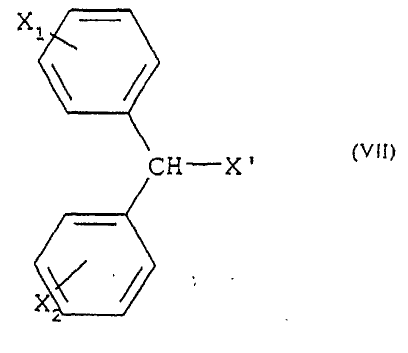 dans laquelle X' représente un atome d'halogène choisi parmi le chlore, le brome ou l'iode et X₁ et X₂ représentent indépendamment un atome d'hydrogène, de fluor, de chlore et/ou de brome.

9. Procédé selon la revendication 8, **caractérisé en ce que** le [2-(1-pipérazinyl)éthoxy)méthyle substitués de formule I dans laquelle R₂ représente un atome d'hydrogène qui est mis en oeuvre est le produit de la réaction de clivage du groupement R₂ d'un [2-(1-pipérazinyl)éthoxy)méthyle substitués de formule I dans laquelle R₂ représente un atome d'hydrogène ou un groupe -COR₄ ou -R₅, où R₄ est choisi parmi les groupes -OR₆ ou -R₇, dans lesquels
R₅ représente un radical allyle ou alkylaryle,
R6 représente un radical alkyle linéaire ou ramifié ayant de 1 à 4 atomes de carbone, un radical halogénoalkyle, alkylaryle, alkylnitroaryle ou alkylhalogénoaryle, et
R₇ représente un halogénoalkyle.

10. Procédé selon la revendication 8 pour la préparation des composés de formule 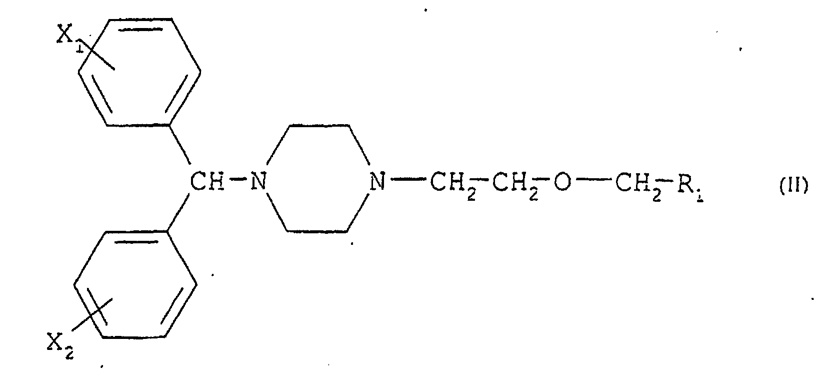 dans laquelle R₁ représente un groupe -CONH₂, -CN, -COOM ou -COOR₃, M étant un métal alcalin et R₃ étant un radical alkyl ayant de 1 à 4 atomes de carbone et X₁ représente un atome de chlore en position 4 et X₂ un atome d'hydrogène, comprenant une étape additionnelle pour la préparation de l'acide 2-[2-[4-[(4-chlorophényl)phénylméthyl)-1-pipérazinyl]éthoxy]acétique et/ou de ses sels pharmaceutiquement acceptables par hydrolyse en milieu aqueux, alcoolique ou hydro-alcoolique et par un acide ou une base.

11. Procédé selon la revendication 8 pour la préparation des composés de formule 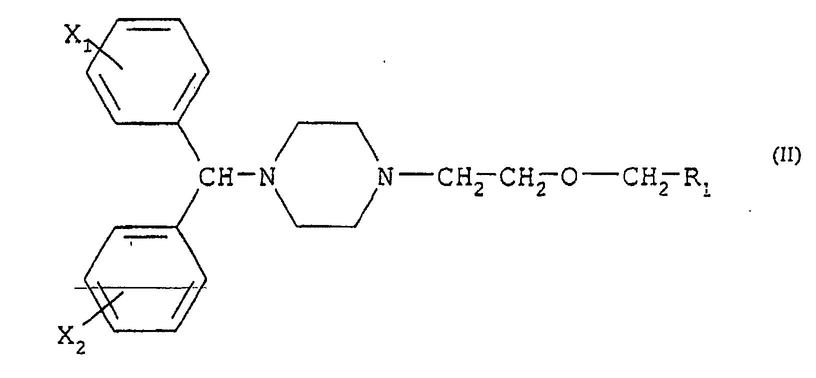 dans laquelle R₁ représente un groupe -CONH₂, -CN, -COCM ou -COOR₃, M étant un métal alcalin et R₃ étant un radical alkyl ayant de 1 à 4 atomes de carbone et X₁ et X₂ représentent chacun un atome de fluor en position 4, comprenant une étape additionnelle pour la préparation de l'acide 2-[2-[4-[bis(4-fluorophényl)phénylméthyl]-1-pipérazinyl]éthoxy]acétique et/ou de ses sels pharmaceutiquement acceptables par hydrolyse en milieu aqueux, alcoolique ou hydro-alcoolique et par un acide ou une base.

## Claims

1. Substituted [2-(1-piperazinyl)ethoxy]methyl compounds of formula 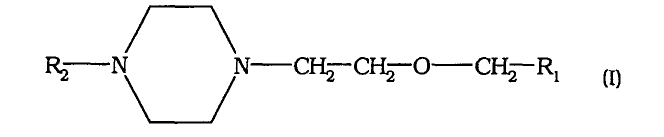 in which
R₁ represents a -CONH₂, -CN, -COOH, -COOM or -COOR₃ group, M being an alkali metal and R₃ being an alkyl radical having from 1 to 4 carbon atoms; and
R₂ represents a hydrogen atom or a group -COR₄ or -R₅, where R₄ is chosen from the groups -OR₆ or -R₇, in which
R₅ represents an allyl or alkylaryl radical,
R₆ represents a linear or branched alkyl radical having from 1 to 4 carbon atoms, a haloalkyl, alkylaryl, alkylnitroaryl or alkylhaloaryl radical, and
R₇ represents a haloalkyl radical.

2. Substituted [2-(1-piperazinyl)ethoxy]methyl compound according to Claim 1, **characterized in that** it is chosen from
2-(1-piperazinyl)ethoxyacetic acid,
2-[2-( 1-piperazinyl)ethoxy]acetamide,
2-(-piperazinyl)ethoxyacetonitrile,
methyl 2-(1-piperazinyl)ethoxyacetate,
ethyl 2-(1-piperazinyl)ethoxyacetate,
2-(4-benzyl-1-piperazinyl)ethoxyacetamide,
2-(4-benzyl-1-piperazinyl)ethoxyacetonitrile,
methyl 2-(4-benzyl-1-piperazinyl)ethoxyacetate,
benzyl 4-(2-carbamoylmethoxyethyl)piperazine-1-carboxylate,
benzyl 4-(2-cyanomethoxyethyl)piperazine-1-carboxylate,
tert-butyl 4-(2-carbamoylmethoxyethyl)piperazine-1-carboxylate,
tert-butyl 4-(2-cyanomethoxyethyl)piperazine-1-carboxylate,
tert-butyl 4-(2-methoxycarbonylmethoxyethyl)piperazine-1-carboxylate,
ethyl 4-(2-carbamoylmethoxyethyl)piperazine-1-carboxylate, and
potassium 2-(4-carboxyethyl-1-piperazinyl)ethoxyacetate.

3. Substituted [2-(1-piperazinyl)ethoxy]methyl compounds according to Claim 1, **characterized in that** the protecting group for the amine function R₂ is a linear or branched alkyl carboxylate group having from 1 to 4 carbon atoms or an alkylaryl group.

4. Process for the preparation of substituted [2-(1-piperazinyl)ethoxy]-methyl compounds according to any of the claims 1 to 3 **characterized in that** a piperazine of formula 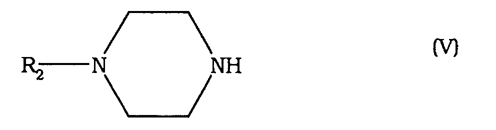 in which R₂ represents a hydrogen atom or a group -COR₄ or -R₅, where R₄ is chosen from the groups -OR₆ or -R₇, in which
R₅ represents an allyl or alkylaryl radical,
R₆ represents a linear or branched alkyl radical having from 1 to 4 carbon atoms, a haloalkyl, alkylaryl, alkylnitroaryl or alkylhaloaryl radical and
R₇ represents a haloalkyl radical,
is reacted with a substituted [2-haloethoxy]methyl compound of formula 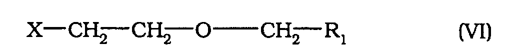 in which R₁ represents a -CONH₂, -CN, -COOH, -COOM or -COOR₃ group. M being an alkali metal and R₃ being an alkyl radical having from 1 to 4 carbon atoms and X represents a halogen atom.

5. Process according to Claim 4, **characterized in that** the halogen atom represented by X is a chlorine or iodine atom.

6. Process according to either of Claims 4 and 5, **characterized in that** the protecting group for the amine function R₂ is a linear or branched alkyl carboxylate group having from 1 to 4 carbon atoms or an alkylaryl group.

7. Use of substituted [2-(1-piperazinyl)ethoxy]methyl compounds of formula 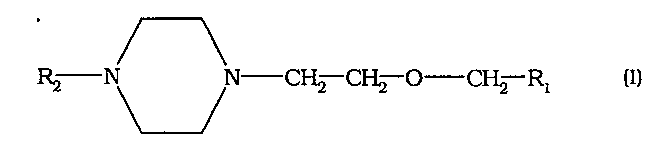 in which
R₁ represents a -CONH₂, -CN, -COOH, -COOM or -COOR₃ group, M being an alkali metal and R₃ being an alkyl radical having from 1 to 4 carbon atoms; and
R₂ represents a hydrogen atom,
for the preparation of compounds of formula 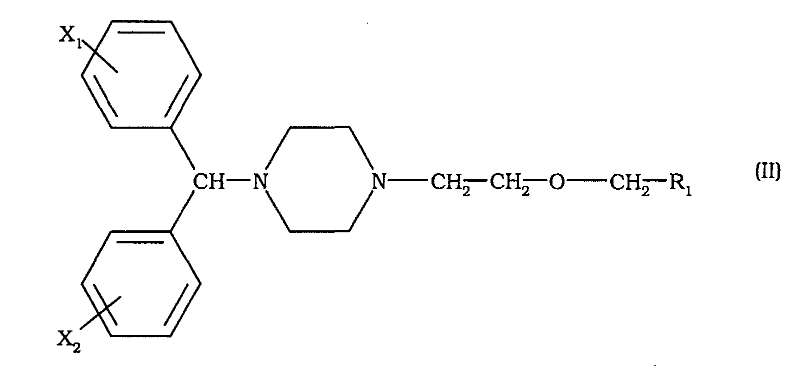 in which R₁ has the same meaning as above in formula (I) and X₁ and X₂ independently represent a hydrogen, fluorine, chlorine and/or bromine atom, by reaction with a diphenylmethyl halide of formula 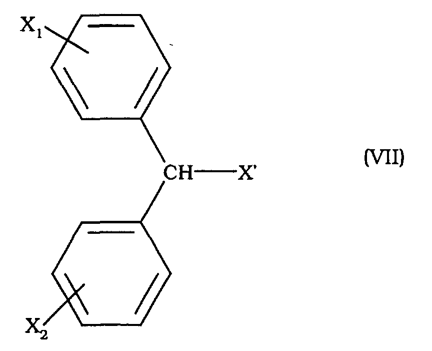 in which X' represents a halogen atom chosen from chlorine, bromine or iodine and X₁ and X₂ have the same meaning as above in formula (II).

8. Process for the preparation of compounds of formula 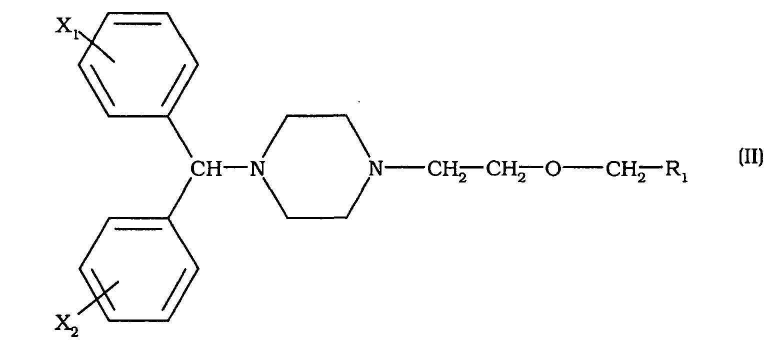 in which R₁ represents a -CONH₂, -CN, -COOH, -COOM or -COOR₃ group, M being an alkali metal and R₃ being an alkyl radical having from 1 to 4 carbon atoms, and X₁ and X₂ independently represent a hydrogen, fluorine, chlorine and/or bromine atom, **characterized in that** a substituted [2-(1-piperazinyl)ethoxy]methyl compound of formula 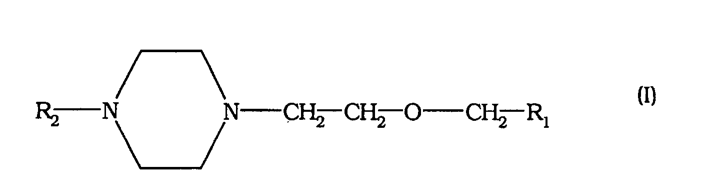 in which
R₁ represents a -CONH₂, -CN, -COOH, -COOM or -COOR₃ group, M being an alkali metal and R₃ being an alkyl radical having from 1 to 4 carbon atoms,
R₂ represents a hydrogen atom,
is reacted with a diphenylmethyl halide of formula 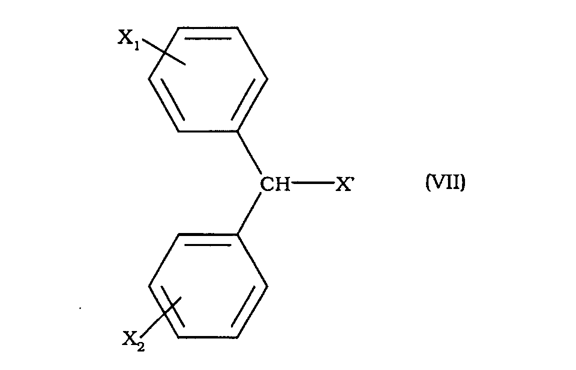 in which X' represents a halogen atom chosen from chlorine, bromine or iodine and X₁ and X₂ independently represent a hydrogen, fluorine, chlorine and/or bromine atom.

9. Process according to Claim 8, **characterized in that** the substituted [2-(1-piperazinyl)ethoxy]methyl compound of formula I, in which R₂ represents a hydrogen atom, which is used is the product of the reaction for the cleavage of the group R₂ of a substituted [2-(1-piperazinyl)-ethoxy]methyl compound of formula I in which R₂ represents a hydrogen atom or a group -COR₄ or -R₅, where R₄ is chosen from groups - OR₆ or -R₇, in which
R₅ represents an allyl or alkylaryl radical,
R₆ represents a linear or branched alkyl radical having from 1 to 4 carbon atoms, a haloalkyl, alkylaryl, alkylnitroaryl or alkylhaloaryl radical, and
R₇ represents a haloalkyl radical.

10. Process according to claim 8 for the preparation of compounds of formula 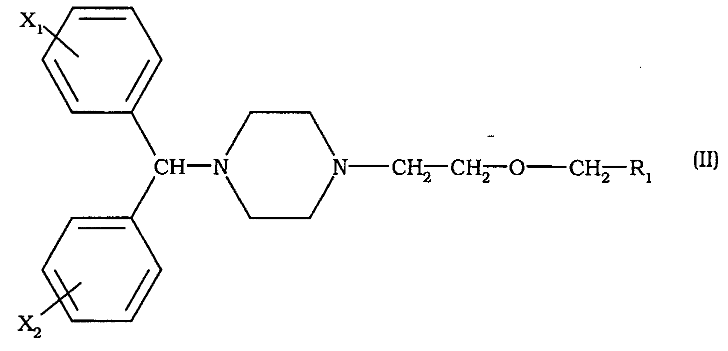 in which R₁ represents a -CONH₂, -CN, -COOM or -COOR₃ group, M being an alkali metal and R₃ being an alkyl radical having from 1 to 4 carbon atoms and X₁ represents a chlorine atom in position 4 and X₂ a hydrogen atom, comprising an additional step for the preparation of 2-[2-[4-[(4-chlorophenyl)phenylmethyl]-1-piperazinyl]ethoxy]acetic acid and/or pharmaceutically acceptable salts thereof, by hydrolysis in aqueous, alcoholic or aqueous-alcoholic medium and using an acid or a base.

11. Process according to claim 8 for the preparation of compounds of formula 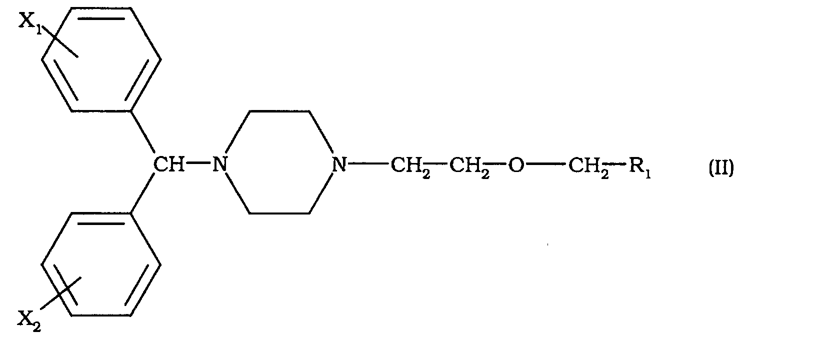 in which R₁ represents a -CONH₂, -CN, -COOM or -COOR₃ group, M being an alkali metal and R₃ being an alkyl radical having from 1 to 4 carbon atoms and X₁ and X₂ each represent a fluorine atom in position 4, comprising an additional step for the preparation of 2-[2-[4-[bis(4-fluorophenyl)phenylmethyl]-1-piperazinyl]ethoxy]acetic acid and/or pharmaceutically acceptable salts thereof, by hydrolysis in aqueous, alcoholic or aqueous-alcoholic medium and using an acid or a base.

## Patentansprüche

1. Substituierte [2-(1-Piperazinyl)ethoxy]methylverbindungen der Formel 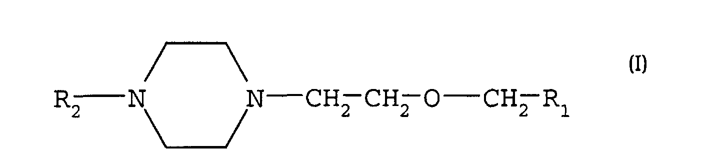 worin
R₁ eine Gruppe -CONH₂, -CN, -COOH, -COOM oder -COOR₃ darstellt, wobei M ein Alkalimetall ist und R₃ ein Alkylrest mit 1 bis 4 Kohlenstoffatomen ist; und R₂ ein Wasserstoffatom oder eine Gruppe -COR₄ oder -R₅ darstellt, worin R₄ unter den Gruppen -OR₆ oder -R₇ ausgewählt ist, in denen
R₅ einen Allyl- oder Alkylarylrest darstellt,
R₆ einen linearen oder verzweigten Alkylrest mit 1 bis 4 Kohlenstoffatomen, einen Halogenalkyl-, Alkylaryl-, Alkylnitroaryl- oder Alkylhalogenarylrest darstellt und
R₇, ein Halogenalkyl darstellt.

2. Substituierte [2-(1-Piperazinyl)ethoxy]methylverbindung gemäß Anspruch 1, **dadurch gekennzeichnet, dass** sie ausgewählt ist unter
2-(1-Piperazinyl)ethoxyessigsäure,
2-[2-(1-Piperazinyl)ethoxylacetamid,
2-( 1-Piperazinyl)ethoxyacetonitril,
2-( 1-Piperazinyl)ethoxyessigsäure-methylester,
2-( 1-Piperazinyl)ethoxyessigsäure-ethylester,
2-(4-Benzyl-1-piperazinyl)ethoxyacetamid,
2-(4-Benzyl-1-piperazinyl)ethoxyacetonitril,
2-(4-Benzyl-1-piperazinyl)ethoxyessigsäure-methylester,
4-(2-Carbamoylmethoxyethyl)-piperazin-1-carbonsäure-benzylester,
4-(2-Cyanmethoxyethyl)-piperazin-1-carbonsäure-benzylester,
4-(2-Carbamoylmethoxyethyl)-piperazin-1-carbonsäure-tert-butylester,
4-(2-Cyanmethoxyethyl)-piperazin-1-carbonsäure-tert-butylester,
4-(2-Methoxycarbonylmethoxyethyl)-piperazin-1-carbonsäure-tert-butylester,
4-(2-Carbamoylmethoxyethyl)-piperazin-1-carbonsäure-ethylester und
2-(4-Carboxyethyl-1-piperazinyl)ethoxyessigsäure Kaliumsalz.

3. Substituierte [2-(1-Piperazinyl)ethoxy]methylverbindungen gemäß Anspruch 1, **dadurch gekennzeichnet, dass** die Schutzgruppe der Aminfunktion R₂ eine lineare oder verzweigte Alkylcarboxylatgruppe mit 1 bis 4 Kohlenstoffatomen oder eine Alkylarylgruppe ist.

4. Verfahren zur Herstellung von substituierten [2-(1-Piperazinyl)ethoxy]-methylverbindungen gemäß einem der Ansprüche 1 bis 3, **dadurch gekennzeichnet, dass** man ein Piperazin der Formel 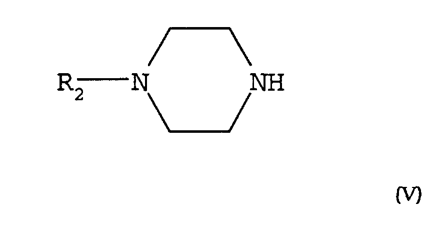 worin R2 ein Wasserstoffatom oder eine Gruppe -COR₄ oder -R₅ darstellt, worin R₄ unter den Gruppen -OR₆ oder -R₇ ausgewählt ist, in denen
R₅ einen Allyl- oder Alkylarylrest darstellt,
R₆ einen linearen oder verzweigten Alkylrest mit 1 bis 4 Kohlenstoffatomen, einen
Halogenalkyl-, Alkylaryl-, Alkylnitroaryl- oder Alkylhalogenarylrest darstellt und
R₇ ein Halogenalkyl darstellt,
mit einer substituierten [2-Halogenethoxy]methylverbindung der Formel
X-CH₂-CH₂-O-CH₂-R1 (VI)
umsetzt, worin R₁ eine Gruppe -CONH₂, -CN, -COOH, -COOM oder -COOR₃ darstellt, wobei M ein Alkalimetall ist und R₃ ein Alkylrest mit 1 bis 4 Kohlenstoffatomen ist und X ein Halogenatom darstellt.

5. Verfahren gemäß Anspruch 4, **dadurch gekennzeichnet, dass** das durch X dargestellte Halogenatom ein Chlor- oder Iodatom ist.

6. Verfahren gemäß einem der Ansprüche 4 oder 5, **dadurch gekennzeichnet, dass** die Schutzgruppe der Aminfunktion R₂ eine lineare oder verzweigte Alkylcarboxylatgruppe mit 1 bis 4 Kohlenstoffatomen oder eine Alkylarylgruppe ist.

7. Verwendung von substituierten [2-(1-Piperazinyl)ethoxy]methylverbindungen der Formel 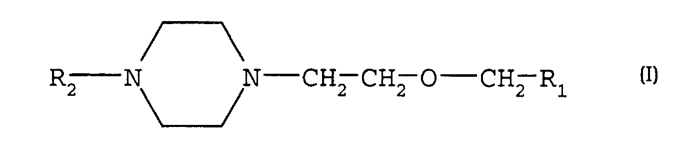 worin R₁ eine Gruppe -COHN₂, -CN, -COOH, -COOM oder -COOR₃ darstellt, wobei M ein alkalimetall ist und R₃ ein Alkylrest mit 1 bis 4 Kohlenstoffatomen ist; und
R₂ ein Wasserstoffatom darstellt, zur Herstellung von Verbindungen der Formel 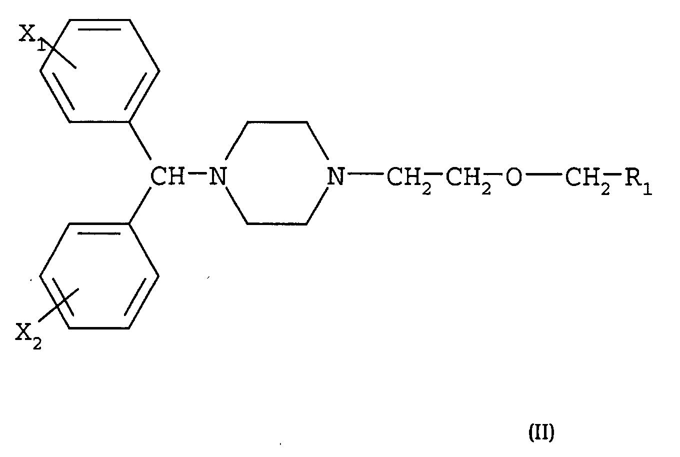 worin R₁ die gleiche Bedeutung wie zuvor in der Formel (I) hat und X₁, und X₂ unabhängig voneinander ein Wasserstoff-, Fluor-, Chlor- und/oder Bromatom darstellen, durch Reaktion mit einem Diphenylmethylhalogenid der Formel 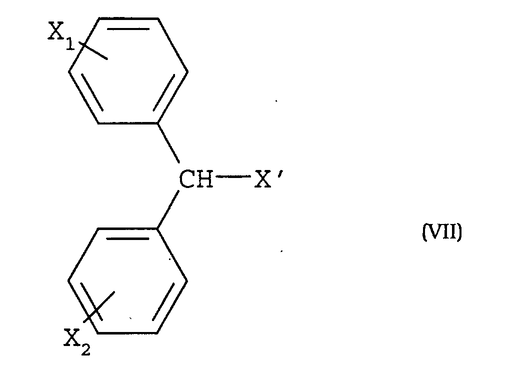 worin X' ein Halogenatom darstellt, das ausgewählt ist unter Chlor, Brom oder lod, und X₁ und X₂ die gleiche Bedeutung wie zuvor in der Formel (II) haben.

8. Verfahren zur Herstellung von Verbindungen der Formel 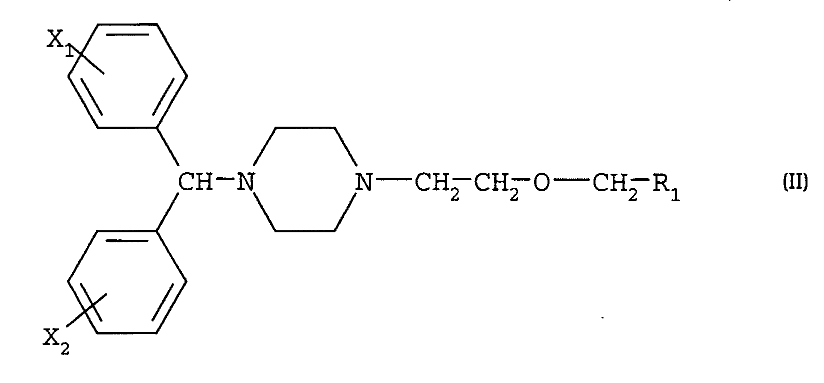 worin R₁ eine Gruppe -CONH₂, -CN, -COOH, -COOM oder -COOR₃ darstellt, wobei M ein Alkalimetall ist und R₃ ein Alkylrest mit 1 bis 4 Kohlenstoffatomen ist, und X₁ und X₂ unabhängig voneinander ein Wasserstoff-, Fluor-, Chlor- und/oder Bromatom darstellen, **dadurch gekennzeichnet, dass** man eine substituierte [2-(1-Piperazinyl)-ethoxy] methylverbindung der Formel 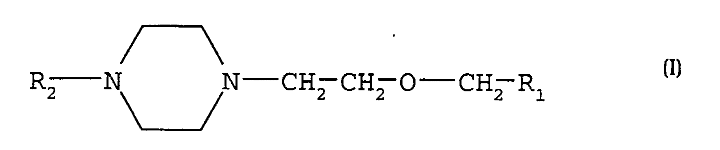 worin
R₁ eine Gruppe -CONH₂, -CN, -COOH, -COOM oder -COOR₃ darstellt, wobei M ein Alkalimetall ist und R₃ ein Alkylrest mit 1 bis 4 Kohlenstoffatomen ist,
R₂ ein Wasserstoffatom darstellt, mit einem Diphenylmethylhalogenid der Formel 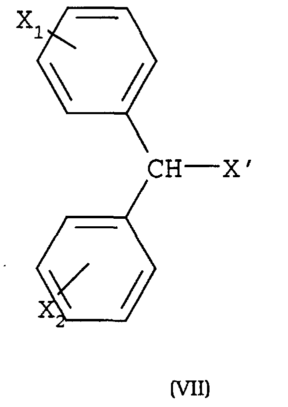 umsetzt, worin X' ein Halogenatom darstellt, das ausgewählt ist unter Chlor, Brom oder lod, und X₁ und X₂ unabhängig voneinander ein Wasserstoff-, Fluor-, Chlor und/oder Bromatom darstellen.

9. Verfahren gemäß Anspruch 8, **dadurch gekennzeichnet, dass** die eingesetzte substituierte [2-(1-Piperazinyl)ethoxylmethylverbindung der Formel I, worin R₂ ein Wasserstoffatom darstellt, das Produkt der Reaktion zur Abspaltung der Gruppe R₂ von einer substituierten [2-(1-Piperazinyl)ethoxy]methylverbindung der Formel I ist, worin R₂ ein Wasserstoffatom oder eine Gruppe -COR4 oder -R5 darstellt, worin R4 unter den Gruppen -OR₆ oder -R₇ ausgewählt ist, in denen
R₅ einen Allyl- oder Alkylarylrest darstellt,
R₆ einen linearen oder verzweigten Alkylrest mit 1 bis 4 Kohlenstoffatomen, einen
Halogenalkyl-, Alkylaryl-, Alkylnitroaryl- oder Alkylhalogenarylrest darstellt und
R₇ ein Halogenalkyl darstellt.

10. Verfahren gemäß Anspruch 8 zur Herstellung der Verbindungen der Formel 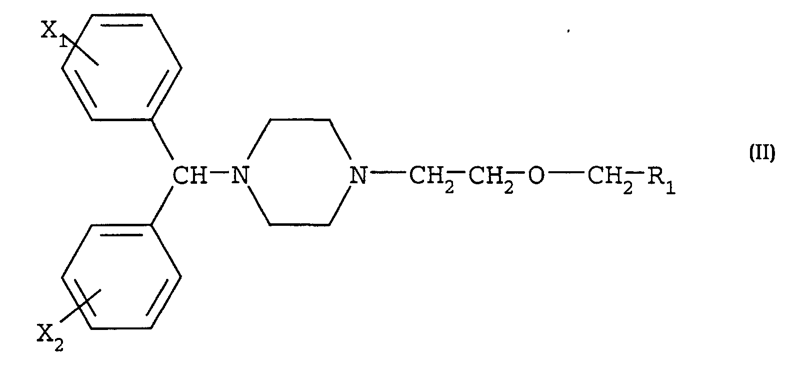 worin R₁ eine Gruppe -CONH₂, -CN, -COOM oder -COOR₃ darstellt, wobei M ein Alkalimetall ist und R₃ ein Alkylrest mit 1 bis 4 Kohlenstoffatomen ist, und X₁ ein Chloratom in Position 4 und X₂ ein Wasserstoffatom darstellt, das einen zusätzlichen Schritt zur Herstellung von 2-[2-[4-[(4-Chlorphenyl]phenylmethyl]-1-piperazinyl]ethoxy]essigsäure und/oder ihren pharmazeutisch annehmbaren Salzen durch Hydrolyse in wässrigem, alkoholischem oder wässrig-alkoholischem Medium und mit einer Säure oder einer Base umfasst.

11. Verfahren gemäß Anspruch 8 zur Herstellung der Verbindungen der Formel 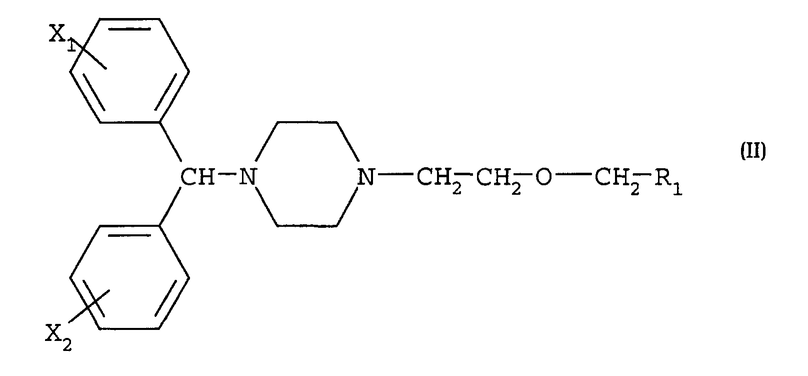 worin R₁ eine Gruppe -CONH₂, -CN, -COOM oder -COOR₃ darstellt, wobei M ein Alkalimetall ist und R₃ ein Alkylrest mit 1 bis 4 Kohlenstoffatomen ist, und X₁ und X₂ jeweils ein Fluoratom in Position 4 darstellen, das einen zusätzlichen Schritt zur Herstellung von 2-[2-[4-[Bis(4-fluorphenyl)phenylmethyl]-1-piperazinyl]ethoxy]essigsäure und/oder ihren pharmazeutisch annehmbaren Salzen durch Hydrolyse in wässrigem, alkoholischem oder wässrig-alkoholischem Medium und mit einer Säure oder einer Base umfasst.
